# EUROPEAN PATENT SPECIFICATION

(11) **EP 2 081 957 B1**
(45) Date of publication and mention of the grant of the patent: **14.08.2019**
(21) Application number: 07816815.0
(22) Date of filing: 13.11.2007
(51) Int. Cl.: A61P 3/10, C07K 14/575, C07K 17/08, C07K 17/06, A61K 38/22, A61K 38/26, A61K 47/65, A61K 47/60

(54) **PEG MODIFIED EXENDIN OR EXENDIN ANALOG AND COMPOSITIONS AND USE THEREOF**
PEG-MODIFIZIERTES EXENDIN ODER EXENDINANALOGON UND ZUSAMMENSETZUNGEN UND ANWENDUNG DAVON
EXENDINE OU ANALOGUE D'EXENDINE MODIFIÉ(E)S PAR PEG, LEURS COMPOSITIONS ET LEUR UTILISATION

(30) Priority: 14.11.2006 CN 200610118326; 23.07.2007 CN 200710138718
(43) Date of publication of application: 29.07.2009
(73) Proprietor: Shanghai Benemae Pharmaceutical Corporation, Zhoupu Town Pudong New Area Shanghai (CN)
(72) Inventor: BAO, Wenchao, Shanghai 200235 (CN); XU, Hongjing, Shanghai 200235 (CN); YU, Gang, Shanghai 200235 (CN); ZUO, Yajun, Shanghai 200235 (CN)
(74) Representative: Gulde & Partner
(86) International application number: PCT/CN2007/003203
(87) International publication number: WO 2008/058461

(56) References cited:
- EP-A1- 1 496 076
- WO-A1-00/66629
- WO-A1-03/076490
- WO-A1-2005/058954
- WO-A2-2004/089280
- WO-A2-2004/089280
- WO-A2-2007/047834
- CN-A- 1 363 559
- CN-A- 1 372 570
- US-A1- 2002 052 443
- US-B2- 6 566 506

## Description

### Background

Glucagon-like peptide-1 (GLP-1) was first discovered in 1987 and identified as a glucose-dependent intestinal secreted hormone peptide. GLP-1 peptide transmits signals through G protein-coupled receptors (GPCR), and stimulates β islet cells to secret insulin to inhibit glucagon secretion, gastric emptying and gastric acid secretion and effects other physiological functions.

The exendins are peptides (J. Biol. Chem. 1999, 265, 20259-20262; J. Biol. Chem. 1992, 267, 7402-7405) found in the salivary secretions of venomous lizards the Gila monster and Heloderma horridum. These exendins, as represented by exendin-4, are highly homologous to GLP-1 [7-36]. Previous researches have discovered that the exendins can bind to GLP-1 receptors and exert similar pharmacological effects, such as stimulating insulin secretion, effectively controlling blood sugar levels after a meal, reducing the glycosylation level of hemoglobin and inhibiting gastric emptying. Testing on animals found that long term use of GLP-1 receptor peptides can effectively reduce the resistance to insulin which may lead to the reversion of diabetes mellitus deterioration. Additionally, it has been found that a number of insulinotropic agonists such as GLP-1 and exendin-4 can stimulate regeneration of β islet cells (Nat. Biotech. 2005, 23, 857-861) and ameliorate nonalcoholic fatty livers (Hepatology 2006, 43, 173-181). These discoveries make such peptides a hot area in the studies of diabetes and adiposis. Wu Dengxi and Sun Yukun (Chinese patent: ZL01112856) have modified exendin-4 and obtained a series of exendin-4 analogs with the same functions as the native exendin-4. Recently, a new exendin based drug, exendin-4 (Byetta®), came into the US market. This drug was jointly developed by Amylin and Eli Lilly and requires two injections per day. The drug has drawn attention in the therapeutic field of diabetes and adiposis. However, clinical researches discovered that about 41% of the patients produced antibodies against exendin-4 after 30 weeks of treating with such drug (Diabetes Care. 2004, 27, 2628-2635).

Protein/peptide drugs typically have shortcomings such as a short half life in blood, poor physical and chemical stability, and prone to in vivo degradation by proteases. As a result, multiple injections of such drugs are required every day, causing lots of pain and inconvenience to patients. How to extend the half life of these drugs has puzzled the biotechnology industry for a long time. Presently, no one has found a universally acceptable solution to this problem.

PEG modification technology emerged in the 1970s and was applied in the technology of making protein/peptide drugs. When certain protein/peptides were modified by linear or branched PEG, the modification may have given the following features to the protein/peptide: (1) an improvement in physical and chemical stability; (2) a decrease in immunogenicity; (3) an increase in resistance to protease degradation; (4) an extension of half life in blood due to the increase in PEG molecular weight leading to reduced kidney clearance; and (5) an improvement in drug solubility and cell membrane penetration. According to studies by A. Yang and K. Precourt, exendin-4 is primarily metabolized through kidney clearance. Therefore, they employed a PEG having a molecular weight in the range of 500 to 20,000 Da to modify exendins (Chinese patent: CN1372570A) to reduce the effect of kidney clearance.

However, a main defect of the PEG technology is that the bioactivity of a modified drug generally drops significantly after the modification. Haim Tsubery et. al. employed 9-hydroxymethy1 -7-sulfofluorene-N-hydroxysuccinimide (FMS) to activate PEG to be coupled with exendin-4, and then the PEG groups were released from the exendin-4 by *in vivo* hydrolysis. Thus the bioactivity of exendin-4 was restored. Although this method provides a solution to the problem of low bioactivity due to PEG modification, un-modified exendin-4 was released after in vivo hydrolysis and the problem of immunogenicity resulting from frequent injections has remained unsolved (J. Biol. Chem. 2004, 279, 38118-38124).

Existing defects of known exendins and exendin analogs include short time intervals between doses, the production of antibodies in patients from long-term injection, and the reduction of bioactivity after modification with PEG. These defects make it hard for exendins and exendin analogs to be applied in practice and require the administration of large dosages of exendins or exendin analogs which severely impedes the application of . exendin technology.

### Summary

The present invention is directed to subject matter as defined in the claims. In certain embodiments, exendin or exendin analogs modified by one or more polyethylene glycol (PEG) molecules or derivatives are provided. In certain embodiments, PEG modified exendins or exendin analogs having one or more PEG derivatives linked to one or more amino acids of the exendins or exendin analogs or derivatives are provided. The PEG derivatives have a branched structure, e.g., as set forth in any one of Formulas (I-I V) described herein. In certain embodiments,
compositions including a PEG modified exendin or exendin analog, methods of making or administering such modified exendins or exendin analogs, and various uses thereof, e.g., for treatment of diabetes, are provided. The PEG modified exendins or exendin analogs exhibit improved and unexpected properties and characteristics, such as, for example, long half life in blood, high bioactivity, and/or low immunogenicity.

### Brief Description of the Drawings

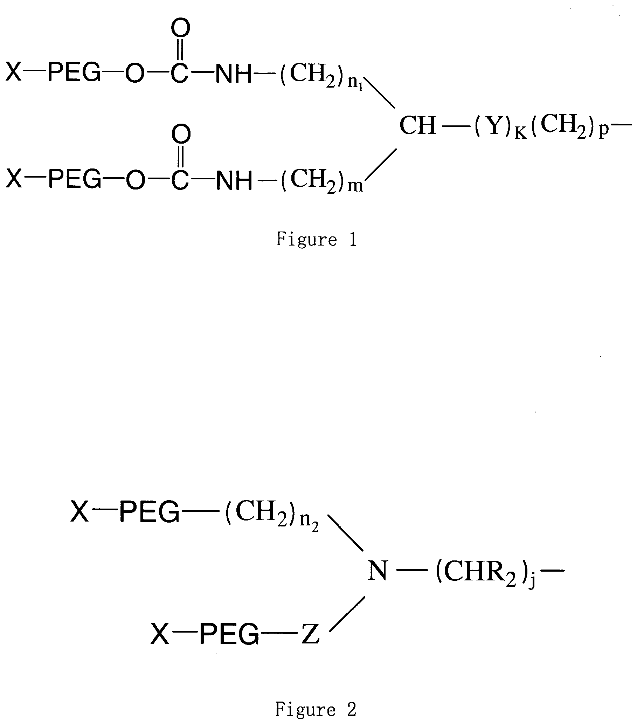

Figure 1 is the structure of Formula I of branched PEG derivatives.
Figure 2 is the structure of Formula III of branched PEG derivatives.

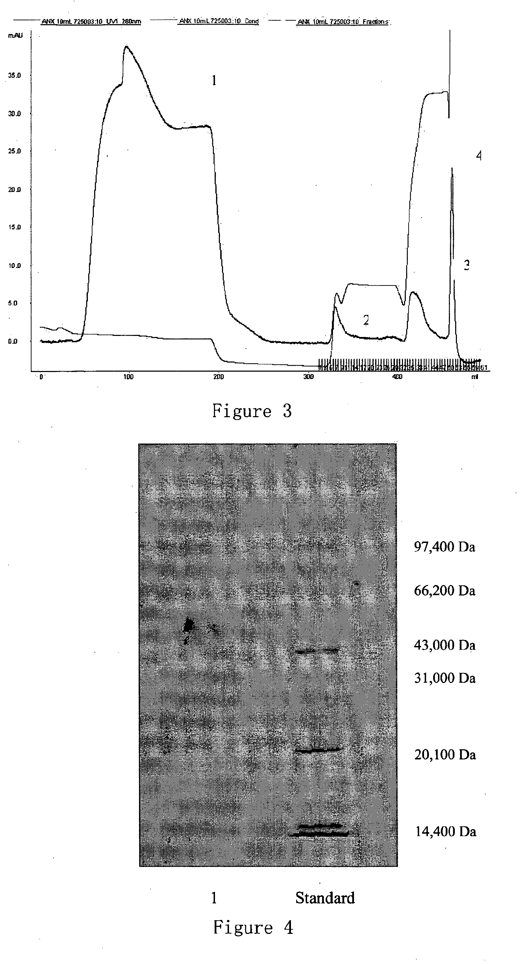

Figure 3 shows the chromatography results from the separation and purification of an exendin-4 analog modified by PEG derivative (Formula IV) having a molecular weight of 40,000 Da (Dalton). Label 1 indicates the absorption peak at the time of sample loading; Label 2 indicates the absorption peak at the time of elution, showing exendin-4 analogs modified by multiple PEG derivatives; Label 3 indicates the absorption peak at the time of elution, showing exendin-4 analogs modified by single PEG derivatives, and Label 4 indicates the absorption peak at the time of elution, showing unmodified exendin-4 analog.
Figure 4 is the electrophoretogram of an exendin-4 analog modified by a single PEG derivative (Formula IV) having a molecular weight of 40,000 Da, wherein lane 1 shows the band of the exendin-4 analog modified by a single PEG derivative.
Figure 5 shows the results of 24-hour drug effect test of exendin-4 analogs modified by a single PEG derivative having molecular weights of 5,000 and 21 ,000 Da, respectively, on animals.
Figure 6 shows the results of 72-hour drug effect test of exendin-4 analogs modified by a single linear or branched (Formula IV) PEG derivative having molecular weights of 21,000 and 40,000 Da on animals.
Figure 7 shows the results of 72-hour drug effect test of exendin-4 analogs modified by single PEG derivatives (Formula II) having molecular weights of 21 ,000 Da (U21 K), 30,000 Da (U30K) and 40,000 Da (U40K), respectively, on animals.
Figure 8 shows the results of 72-hour drug effect test of exendin-4 analogs modified by single PEG derivatives (Formula IV) having molecular weights of 21 ,000 Da (Y21 K), 30,000 Da (Y30K) and 40,000 Da (Y40K), respectively, on animals.
Figure 9 shows the results of 72-hour drug effect test of exendin-4 analogs modified by single linear PEG derivatives having molecular weights of 21 ,000 Da (L21 K) and 30,000 Da (L30k), respectively, on animals.
Figure 10 shows the results of 72-hour drug pharmacokinetics test of exendin-4 analogs modified by single PEG derivatives (Formula II) having molecular weights of 30,000 Da (U30K) and PEG derivatives (Formula IV) having molecular weights of 40,000 Da (Y40K), respectively.

### Detailed Description

### PEG Modification

The following description of the invention is merely intended to illustrate various embodiments of the invention.

In certain embodiments, exendins or exendin analogs modified by one or more PEG derivatives are provided. In certain embodiments, novel PEG modifications of exendins and/or exendin analogs are employed to make PEG modified exendins or exendin analogs which exhibit improved and unexpected properties and characteristics, such as, for example, long half life in blood, high bioactivity, and/or low immunogenicity.

Different chemical structures of PEG derivatives may have different effects on the bioactivity of the peptides modified by such PEG derivatives or molecules. PEG derivatives may have linear or branched structures. The branched structures include, e.g., double branched-chains as well as multiple branched-chains. The PEG derivatives having double branched-chains include, for example, without limitation, the structures set forth in Formulas I, II, III and IV below.

In certain embodiments, the branched PEG derivatives may include, for example, without limitation, the structure set forth in Formula I below:

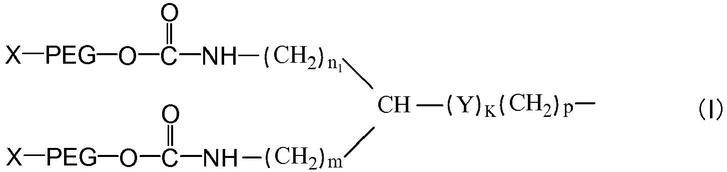

Wherein, X is hydrogen or a protecting group. The protecting group may be any group which can react with a free hydroxyl group of the PEG derivative, and at the same time can prevent its further reaction with other groups. Examples of protecting groups include, for example, without limitation, alkyl groups such as methyl, dimethyl, ethyl, propyl, and isopropyl.

Furthermore, PEG is -O(CH₂CH₂O])_{q}-, q is a positive integer; n₁ is an integer from 0-5; m is an integer from 0-5; Y is O, S, SO, SO₂ or NR₁, wherein R₁ is hydrogen or substituted or unsubstituted (C₁-C₈) alkyl group or substituted or unsubstituted cycloalkyl group; k is 0 or 1 ; and p is an integer from 0-6. A PEG having the structure set forth in Formula I can be synthesized by conventional chemical methods. For example, a synthesis method for PEG having the structure set forth in Formula I is described in U. S. Patent No.: 6,566,506.

In certain embodiments, a PEG derivative used herein which comprises the branched structure of Formula I has the specific structure set forth in Formula II below:

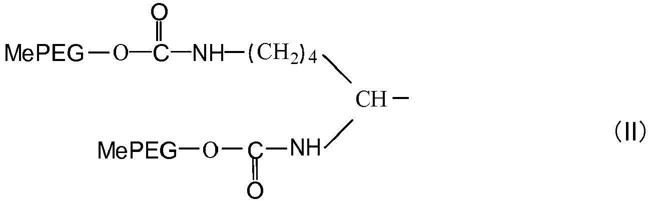

Wherein, PEG is -O(CH₂CH₂O)_{q}-, q is a positive integer, and Me is a methyl group. In certain embodiments, a branched-chain PEG derivative has the structure of Formula III below:

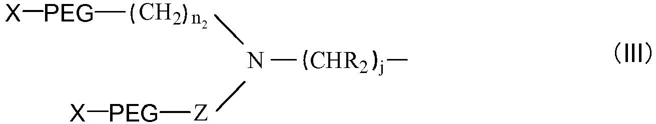

Wherein, X is hydrogen or a protecting group; PEG is -O(CH₂CH₂O)_{q}-, q is a positive integer; n₂ is an integer from 0-10; Z is: (CH₂)ᵢ, (CH₂)ᵢOCO, (CH₂)ᵢNHCO, or (CH₂)ᵢCO, wherein i is an integer from 0-10; R₂ is hydrogen, substituted or unsubstituted (C₁-C₁₂) alkyl group, substituted aryl, aralkyl or heteroalkyl group; and j is an integer from 1-12. The above mentioned PEG derivative structure of Formula III can be synthesized by conventional chemical methods, one of which is provided in the Chinese Patent No. ZL03801105.0.

In certain embodiments,, a PEG derivative used herein which comprises the branched structure of Formula III has the structure set forth in Formula IV below: 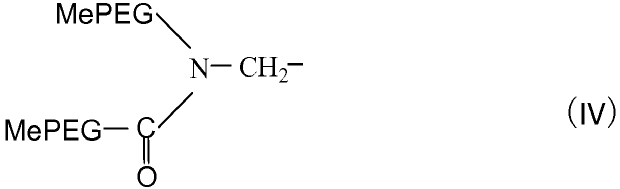 Wherein, PEG is -O(CH₂CH₂O)_{q}-, q is a positive integer, and Me is a methyl group. The molecular weights of PEG derivatives may affect the bioactivity of PEG modified peptides.

In certain embodiments, the PEG derivatives have branched structures with molecular weights in the range of higher than 20,000 Da to about 50,000 Da, or higher than 20,000 Da to about 45,000 Da, or higher than 20,000 Da to about 40,000 Da.

The PEG modification of exendins or exendin analogs is achieved by linking the activated PEG derivatives to side chain of an amino acid residue or the N-terminus or C-terminus of amino acids of the exendins or exendin analogs. For example, PEG derivatives containing different activation groups can bind to different side chains, the N-terminus or C-terminus of amino acids, or to a specific amino acid. Amino acid groups that can be chemically bound to activated PEG derivatives include but are not limited to the N-terminus [alpha]-amino groups, the lysine residue side chain ε-amino groups, imino groups on histidine residue side chain imidazolyl group of peptides, the carboxyl terminal of peptides, side chain carboxyl groups of aspartic acid and glutamic acid, side chain hydroxyl groups of serine and threonine, side chain mercapto groups of cysteine, etc. In general, such chemical binding is achieved through eletrophilic and nucleophilic reactions. For example, the linkage of PEG derivatives activated by N-hydroxyl succinimide to α-amino groups of N-terminus, lysine side chain ε-amino groups and free amino groups such as imino groups on histidine side chain imidazolyl groups are created through such reactions. Additionally, PEGs containing aldehyde activation groups can be specifically linked to the N-terminus of peptides by reductive alkylation. Indeed, methods of linking PEG derivatives to exendins or exendin analogs by using various activation groups, and the PEG modified exendins or exendin analogs produced thereof are provided in certain embodiments. PEG derivatives with various activation groups can be purchased from commercial providers, or synthesized using conventional methods known in the relevant technical field.

In certain embodiments, exendins or exendin analogs coupled with one or more PEG derivatives are provided where the number of PEG derivatives coupled to the exendins or exendin analogs is dependent upon the number of free radical groups on the exendins or exendin analogs, the activation groups of PEG derivative, and/or the PEG derivative's molecular weight. Generally, the more free radical groups that the exendins or exendin analogs have, the more PEG derivatives will be linked thereto. Also generally, the larger the activated PEG derivative's molecular weight is, the less PEG derivative will be linked to the peptides. In certain embodiments, the exendins or exendin analogs are modified by one, two, three or four PEG derivatives. Preferably, an exendin or exendin analog is modified by one or two PEG derivatives.

Mixtures generated by binding PEG derivatives to exendins or exendin analogs can be effectively separated by conventional means, for example, ion exchange separation, gel filtration separation, or reversion. phase chromatography. The ion exchange chromatography may include anion and cation exchange chromatography. Different ion exchange methods may have different effects on the separation and purification results. Since different exendins or exendin analogs have different numbers and kinds of amino acids, and thus have different molecular weights, the ultimate molecular weights of exendins or exendin analogs modified by PEG derivatives are the total molecular weights of the PEG derivatives and the exendins or exendin analogs. After the processes of separation, purification and buffer solution substitution, the PEG modified exendins or exendin analogs may be further processed to make pharmaceutical compositions.

### Exendins or Exendin Analogs

Exendins or exendin analogs herein refer to peptides or peptide derivatives having an amino acid sequence homologous or identical to a portion or the entire sequence of native exendins. Exendins or exendin analogs can bind to GLP-1 receptors and stimulate a cascade of cell signal transmissions. Such peptides can be obtained through solid phase chemical synthesis or genetic engineering, followed by separation and purification.

Exendins or exendin analogs herein include but are not limited to the native exendin-3 and exendin-4. The native exendin-3 has the following amino acid sequence:

The native exendin-4 has the following amino acid sequence:

In certain embodiments, the exendins or exendin analogs include but are not limited to the peptide analogs obtained by amino acid substitutions, additions or deletions of the native exendin-3 and exendin-4 amino acid sequences. The amino acids substitutions of Exendin-3 and Exendin-4 include substitutions with natural amino acids as well as unnatural amino acids. Unnatural amino acids include but are not limited to azetidinecarboxylic acid, 2-amino-hexanedioic acid, 3-amino-hexanedioic acid, β-lactamic acid, aminopropionic acid, 2-aminobutanoic acid, 4-aminobutanoic acid, 6-aminohexanoic acid, 2-aminoheptanoic acid, 2-amino-2-methylpropanoic acid, 3-amino-2-methylpropanoic acid, 2-aminoheptanedioic acid, 2-amino-3,3-dimethylbutanoic acid, desmonsine, 2,2-diaminoheptanedioic acid, 2,3-diaminopropanoic acid, N-ethylglycine, N-ethylasparagine, homoproline, hydroxylysine, allo-hydroxylysine, 3-hydroxyproline, 4-hydroxyproline, isodesmonsine, alloisoleucine, N-methylalanine, N-methylglycine, N-methylisoleucine, N, N-methylpentylglycine, N-methylvaline, naphthalanine, norvaline, norleucine, ornithine, pentylglycine, pipecolic acid and thioproline. Preferentially, exendin analogs include one, two, three, four or five amino acid substitutions.

In certain embodiments, the exendin analogs include peptide analogs obtained by adding or subtracting one or more amino acids to or from the native exendin-3 or exendin-4 amino acid sequences. Preferably, the exendin analogs include peptide analogs obtained by adding or removing one to twenty amino acids to or from the native exendin-3 or exendin-4 amino acid sequences. In certain embodiments, the exendin analogs include peptide analogs obtained by adding or removing one to fifteen amino acids to or from the native exendin-3 or exendin-4 amino acid sequences or by adding or removing one to ten amino acids to or from the native exendin-3 or exendin-4 amino acid sequences or by adding or removing one to nine amino acids to or from the native exendin-3 or exendin-4 amino acid sequences.

Exendin analogs may have reversible or irreversible chemical blocking or modification on their N-terminus, C-terminus, or side chains. For example, the C-terminus of an exendin or exendin analog may be amidated.

In certain embodiments, the exendins or exendin analogs comprise amino acid sequences of SEQ ID NOs: 1 -265. Preferably, exendins or exendin analogs comprise amino acid sequences of SEQ ID NOs: 1 -2 or amino acid sequences of SEQ ID NOs: 3-229 or amino acid sequences of SEQ ID NOs: 230-265.

The activity of PEG modified exendins or exendin analogs described herein can be tested at the cell level according to the assay published by Gong Qiuhong et. al. (Chinese Journal of Incretion and Metaboly, 2004, 20, 559-560). Briefly, the assay is conducted by adding glucose and PEG modified exendins or exendin analogs of various concentrations to INS-1 cells, incubating for 4 hours, detecting the amount of insulin in the supernatant using radioimmunoassay and finally analyzing the amount of insulin in INS-1 cells using RT-PCR technology in a semi-quantitative assay. By using the aforesaid method, large scale screening of the activity of PEG derivative modified exendins or exendin analogs can be achieved. Such screening can also be achieved by testing the change in blood sugar levels in a mouse (e.g. C57 or db/db mouse) at different time points after doses.

### Pharmaceutical Compositions and Uses Thereof

In certain embodiments, pharmaceutical compositions or compounds including PEG modified exendins or exendin analogs as described herein are provided. The PEG derivative modified exendins or exendin analogs described herein may react with various inorganic and organic acids or alkali to form salt. Such salts include salts prepared with organic and inorganic acids, wherein the organic and inorganic acids include but are not limited to hydrochloric acid, hydrobromic acid, sulfuric acid, phosphoric acid, trifluoroacetic acid, acetic acid, formic acid, methanesulfonic acid, toluenesulfonic acid, maleic acid, fumaric acid and camphorsulfonic acid. Salts prepared with alkali include but are not limited to ammonium salts, alkali metal salts (e.g., sodium and potassium salts), and alkali earth metal salts, (e.g. calcium and magnesium salts). Preferred salts include, e.g., acetate salts, hydrochloride salts and trifluoroacetic salts. The salts may be prepared by conventional methods. For example, such salts may be prepared by reacting the exendins or exendin analogs with one or more equivalents of the appropriate acid or alkali in a solvent or medium in which the resulting salt is insoluble or in a solvent such as water, that is removable by vacuum or by freeze-drying, or by exchanging the ions of an existing salt for another ion on a suitable ion exchange resin.

In certain embodiments, PEG modified exendins or exendin analogs as described herein can also be prepared as pharmaceutically acceptable salts (e.g., salt resulting from an addition reaction of acid) and/or complexes thereof. The preparation of such salts can facilitate the pharmacological use by altering the physical or chemical characteristics of a composition without preventing the composition from exerting its physiological effect. Examples of useful alterations in physical properties include lowering the melting point to facilitate transmucosal administration or increasing the solubility to facilitate the administration of higher concentrations of the drug.

Pharmaceutically acceptable salts include, for example, acid addition salts such as those containing sulfate, hydrochloride, phosophate, sulfamate, acetate, citrate, lactate, tartrate, methanesulfonate, ethanesulfonate, benzenesulfonate, p-toluenesulfonate, cyclohexylsulfamate and quinate salts. Pharmaceutically acceptable salts can also be prepared with various organic and inorganic acids such as, e.g., hydrochloric acid, sulfuric acid, phosphoric acid, sulfamic acid, acetic acid, citric. acid, lactic acid, tartaric acid, malonic acid, methanesulfonic acid, ethanesulfonic acid, benzenesulfonic acid, p-toluenesulfonic acid, cyclohexylsulfamic acid, and quinate. Such salts may be prepared by reacting the exendins or exendin analogs with one or more equivalents of the appropriate acid or alkali in a solvent or medium in which the resulting salt is insoluble or in a solvent such as water which is removable by vacuum or by freeze-drying, or by exchanging the ions of an existing salt for another ion on a suitable ion exchange resin.

Carriers or excipients can also be used to facilitate the administration of a composition to a subject. Examples of carriers and excipients include, e.g., calcium carbonate, calcium phosphate, various sugars (e.g. lactose, glucose or sucrose), or various types of starch, cellulose derivatives, gelatin, vegetable oils (e.g. sesame oil, peanuts oil, olive oil), polyethylene glycols and physiologically compatible solvents. Compositions or pharmaceutical compositions can be administered by different routes including, e.g., intravenous, intraperitoneal, subcutaneous and intramuscular, oral, topical and transmucosal administration.

If desired, solutions of the compositions of the invention may be thickened with a thickening agent such as methylcellulose. They may be prepared in an emulsified form (e.g. water in oil or oil in water). Any of the pharmaceutically acceptable emulsifying agents known in the art may be employed including, e.g., acacia gum powder, a non-ionic surfactant (e.g. Tween), or an ionic surfactant (e.g. alkali polyether alcohol sulfates or sulfonates such as Triton).

Compositions may be sterilized by conventional sterilization techniques or filtering. Compositions may contain pharmaceutically acceptable auxiliary substances which have approximate physiological conditions as required, such as pH buffering agents. Useful buffers include, for example, sodium acetate/acetic acid buffers. A form of suppository or preparation with slow release function may be used so that pharmaceutically effective amounts of the preparation remain in the bloodstream over many hours or days following transdermal injection or delivery.

The desired isotonicity may be obtained by using sodium chloride or other pharmaceutically acceptable agents such as glucose, boric acid, sodium tartrate, propyleneglycol, polyols (e.g. mannitol and sorbitol), or other inorganic or organic solvents. Sodium chloride is preferred for buffers containing sodium ion.

In one embodiment, for a patient with a body weight of about 70kg, the effective dosage of the compound will be in the range of about 0.01 or about 0.03 to about 5 mg per day, preferably about 0.01 or about 0.5 to about 2 mg per day, or more preferably about 0.01 or about 0.1 to about 1 mg per day, administered in one or more doses. The exact dose may be determined by the attending physician and is dependent upon whether the specific compound lies within the above quoted ranges, as well as upon the age, weight and symptom of the individual patient.

In certain embodiments, a PEG modified exendin or exendin analog as described herein may be administered to a subject to treat diabetes mellitus. In certain embodiments, administration of the compound should begin immediately at the time diabetes mellitus symptoms manifest or right after diabetes mellitus is diagnosed. Optionally, in other embodiments, the compound may be administered before symptoms manifest as a preventative treatment.

Although the compounds are typically used to treat human patients, they may be used to treat similar or identical diseases in other vertebrates as well, such as other primates, farm animals (e.g. swine, cattle and poultry), animals or pets for sports (e.g. horses, dogs and cats).

### Examples

A linear PEG derivative with a molecular weight of 5,000 Da was purchased from Sigma-Aldrich Corporation. Other PEG derivatives used herein were purchased from Beijing JenKem Technology Co., Ltd.

A, B Double-Pump AKTA purifiers used in the ion exchange experiments, and other columns and packing were purchased from General Electric. Relevant reagents were purchased from Sigma-Aldrich. The native exendin-4 and its analogs used in Examples 3-9 were purchased from Chengdu Shennuo Science and Technology Co. Ltd.

### Example 1. Solid phase synthesis of exensin-4 analogs

Example 1 presents a method of solid phase synthesis of the exendin-4 analog having the following amino acid sequences:

### (1)Amino acid residues:

| | |
|---|---|
| Fmoc-L-Ala-OH | Fmoc-L-Lys(Boc)-OH |
| Fmoc-L-Asn(Trt)-OH | Fmoc-L-Met-OH |
| Fmoc-L-Asp(OtBu)-OH | Fmoc-L-Phe-OH |
| Fmoc-L-Gln(Trt)-OH | Fmoc-L-Pro-OH |
| Fmoc-L-Glu(OtBu)-OH | Fmoc-L-Ser(tBu)-OH |
| Fmoc-L-Gly-OH | Fmoc-L-Thr(tBu)-OH |
| Fmoc-L-His(Trt)-OH | Fmoc-L-Trp-OH |
| Fmoc-L-Ile-OH | Fmoc-L-Tyr(tBu)-OH |
| Fmoc-L-Leu-OH | Fmoc-L-Val-OH |

Wherein:
Fmoc refers to 9-Fluorenylmethoxycarbonyl;
BOC refers to tert-butyloxycarbonyl;
Trt refers to triphenylmethyl;
OtBu refers to tert-butyl ester;
tBu refers to tert-butyl.

### (2) Apparatus and Reagents for the Synthesis

Peptide syntheses were conducted using a Peptide Synthesizer 433A (Applied Biosystem, USA).

Reagents used for these syntheses were N-Methylpyrrolidone, methylene dichloride, hexahydropyridine, methanol, dimethylaminopyridine / DMF, N, N-diisopropylethylamine / NMP, HBTU 100mmole/0.5M HOBT in DMF, N, N-Dicyclohexylcarbodiimide / NMP.

Wherein:
DMF refers to N, N-Dimethylformamide,
NMP refers to N-Methylpyrrolidone,
HOBT refers to 1-Hydroxybenzotriazole,
HBTU refers to 2-1H-benzotriazole-y1-1,1,3,3-tetramethyl-Uroniumhexa-fluorophosphate.

### (3) Procedures

### a. Synthesis

The quantities of the reagents used in the procedures below were based on a synthesis scale of 0.25mmol. 0.25g of HMP resin was weighed and placed in the synthesizer's reactor vessel. 1 mmol of various amino acids having protecting groups, were weighed and arrayed in the synthesizer in the order of the amino acid sequence of the desired insulinotropic peptide derivative from the C-terminus to the N-terminus. At a room temperature of 25°C, reactions for removing Fmoc protection, activating a residue and attaching the activated residue to HMP resin were automatically performed under the control of a computer program. Such reactions were repeated until the whole peptide was synthesized. After completion of the synthesis, the resin attached with the synthesized peptide having protecting groups to its amino acid side chains was air dried in the peptide synthesizer and then weighed.

### b. Removal of protecting groups and detachment of resin

The resin attached with the synthesized peptide having protecting groups was placed in a plugged Ehrlenmeyer flask, and the cleavage reagent as shown below was added.

| Reagent | Amount |
|---|---|
| Water | 0.50 ml |
| Methyl-phenoxide | 0.50 ml |
| Phenol | 0.75 g |
| Mercaptoethanol | 0.20 ml |
| Trifluoroacetic acid (TFA) | 10.0 ml |

This reaction was carried out at constant temperature of 30°C for six hours with constant stirring. After that, the mixture was filtered, the aqueous filtrates were collected, and the resin was rinsed with a small amount of Trifluoroacetic acid (TFA). The rinsing solution was mixed with the collected aqueous filtrates. Then the mixture was precipitated with ether and the precipitates were rinsed with a small amount of ether.
The precipitates were dried in the drying apparatus to obtained the crude product.

### c. Separation and purification by HPLC and Lyophilization

The crude product was separated and purified by preparative HPLC, and then lyophilized to obtain the final product. The molecular weight of the product was analyzed using Chromatography and mass spectrometry. The theoretical molecular weight of the synthesized peptide was 4300.6 and the actual molecular weight measured was 4316.7.

Similarly, the above method can be used by a technician skilled in the art to synthesize other exendins or exendin analogs.

### Example 2. Preparation of an exendin-4 analog by genetic engineering techniques

This example describes a method to make the exendin-4 analog having the following amino acid sequences by a genetic engineering method:

A. The following gene fragments were synthesized based on the amino acid sequences of the exendin analog to be produced:
(1)
(2)
(3)
(4)

### B. Cloning

Ligation: Two test tubes were taken. Gene fragments (1) and (4) were added and mixed in one tube. Gene fragments (2) and (3) were added and mixed in the other tube. Polynucleotide kinase buffer, polynucleotide kinase, and adenosine triphosphates (ATP) were added to each tube. The reaction mixtures were incubated at 37°C for 60 minutes to phosphorylated the 5' end of the gene fragments. The two tubes were placed in a 95 °C water bath to incubate for 10 minutes. Then the tubes were naturally cooled down to room temperature. T4 ligase buffer and T4 ligase were added to each tube, and the mixtures were incubated overnight at 16°C to allow ligation of the gene fragments.

Plasmid: A plasmid containing a Lac promoter (a temperature control promoter PL, or a Tac promoter) was digested with restriction endonucleases EcoR I and Hind III and extracted with phenol / chloroform solution. The mixture was centrifuged and the aqueous phase was collected. The aqueous phase was extracted with chloroform and centrifuged for three times. The resulting aqueous phase was precipitated with isopropanol, centrifuged and air dried.

The digested plasmid and the ligated gene fragment were mixed together. T4 ligase buffer and T4 ligase were added to the mixture and incubated at room temperature for 3 to 4 hours.

Culturing of host cells: E. Coli JM103 were incubated at 37°C in LB culture solution containing 10g of peptone, 5g of yeast extract and 5g of NaCl for 4 hours with shaking. The bacteria cultures were centrifuged and the precipitates were treated with CaCl₂ solution and kept at 4°C for further use.

Transformation: The cloned plasmid was transformed into E. Coli JM103 host cells. The transformed bacteria cells were incubated in an ice bath for 30 minutes, and then incubated at 42°C for 2 minutes. The bacteria cells were spread out on an agar plate containing Ampicillin and were incubated overnight at 37°C. Colonies grown on the agar plate were selected as the positive clones containing the recombinant plasmids.

### C. Fermentation

The selected host bacteria strain containing the desired plasmid was incubated with shaking in LB culture solution. 0.5mM of Isopropyl beta-D-thiogalactopyranoside (IPTG) was added to the culture solution to induce expression of the desired peptide. After overnight incubation, the bacteria cells were harvested by centrifugation. The expressed peptides were identified by polyacrylamide gel electrophoresis (PAGE) containing 12% SDS.

### D. Inclusion bodies

Ten bottles, each containing 300ml of bacteria cultures, were incubated with shaking under the conditions described above. After induction of protein expression, lysis solution (20mM of phosphoric acid buffer containing 1% NaCl, pH 7.5) and lysozyme were added to the culture solution and incubated at 30°C for 30 minutes and then centrifuged to collect the precipitates. The collected precipitates were treated with 6M guanidine hydrochloride (Gu.HCl) to dissolve the inclusion bodies. The solution was centrifuged, and the resulting supernatant was dialyzed to remove the Gu.HCl. The precipitates resulting from the dialysis were rinsed three times with 20mM phosphoric acid buffer (pH 7.5) containing 1% NaCl and 0.1% Tween 80 to obtain the inclusion bodies.

### E. Degradation

The inclusion bodies were dissolved in 8 M urea solution. Then hydrochloric acid and cyanogen bromide were added to the solution. The final concentration of hydrochloric acid in the solution was 50mM. The solution was stirred in dark and under nitrogen gas protection for 2 hours to break up the inclusion bodies. HPLC analysis was used to monitor the process.

### F. Purification

After the inclusion bodies were broken up, the crude product was obtained through Sepharose G-25 chromatography. The crude product was further purified by HPLC to obtain the final product. Similar to the product synthesized through chemical process, the experimental molecular weight of the obtained peptide measured by mass spectrometry was consistent with its theoretical molecular weight.

### Example 3. Exendin-4 modified with linear methoxy-polyethylene glycol (mPEG) derivatives (Mf: 5,000 Da)

1.0mg of exendin-4 was put into each of three tubes, and dissolved in phosphoric acid buffer having different pH values, respectively. 5.8mg of N-Hydroxysuccinimide-activated mPEG (Mf: 5,000 Da) was added to each tube. The mixtures were placed on a shaking table for an hour at room temperature. The resulting products were analyzed to measure the amount of the un-modified exendin-4 using Agilent 1100 HPLC (Analysis conditions are: 0.1% phosphoric acid as solvent A and 0.1% phosphoric acid + 80% acetonitrile as Solvent B, gradient: 35%-70% solvent B/25 minutes). The results are as follows:

**Effects of different pH values on exendin-4 pegylation**

| pH values | Un-modified Exendin-4 |
|---|---|
| 6.5 | 52% |
| 7.5 | 21% |
| 8.5 | 4% |

A solution (pH value 3-4) containing mPEG modified exendin-4 was obtained after acetonitrile was removed from the solution obtained by reverse phase separation above.

### Example 4. Exendin-4 analogs modified with linear mPEG derivatives (Mn: 21,000 Da)

1.0mg exendin-4 analog was mixed with 2.0 ml of phosphoric acid buffer (pH 4.5) containing NaBH₃CN. 5.0mg of aldehyde activated mPEG derivatives with a molecular weight of 21,000 Da was mixed with the exendin-4 analog and placed on the shaking table reacting overnight at room temperature. The reaction solution was purified by Agilent 1100 Chromatography using Zorbax SB-300 Semi-Preparative Column (Analysis conditions are: 0.1% phosphoric acid as solvent A and 0.1% phosphoric acid + 80% acetonitrile as Solvent B, gradient: 35%-70% solvent B/25 minutes). An exendin-4 analog modified by mPEG at the N-terminus was thereafter obtained.

After the acetonitrile was removed from the solution collected above with a rotary evaporator, a desalting column was used to exchange the buffer of the modified exendin-4, which was then dissolved in the phosphoric acid buffer (pH 7.0-8.0) with 0.001-1.0 % (w/v).3-methylphenol.

### Example 5. Exendin-4 analog modified with mPEG derivatives (Mn: 40,000 Da) having the structure of Formula IV

1.0mg of an exendin-4 analog (SEQ ID NO: 251) was added into 4.0ml phosphoric acid buffer (pH 7.0). 280mg of N-Hydroxysuccinimide-activated mPEG derivative (Formula IV) with a molecular weight of 40,000 Da was mixed with the exendin-4 analog prepared above, and placed on the shaking table reacting for an hour at room temperature. After that, the reaction mixture was diluted with 200ml Bristris buffer (pH 7.0) in a 250ml beaker for further use.

A DEAE FF or ANX FF anion exchange column was equilibrated with Bistris buffers (pH 7.0). The diluted reaction mixture was loaded to the anion exchange column to allow the target substance to absorb onto the anion exchange resin. Single or multiple modified exendin-4 analogs were eluted through linear gradient elution of NaCl at different concentrations, and the eluent was collected with a fraction collector. The un-modified exendin-4 peptide was then eluted by using 1M NaCl. The eluent fractions from the separation and purification process was shown in Figure 3, wherein labels 2, 3 and 4 indicated the elution peaks of multiple mPEG modified exendin-4 analog, single mPEG modified exendin-4 analog and un-modified exendin-4 analog, respectively. The modified exendin-4 analog was run by gel electrophoresis with SDS-PAGE. The molecular weight of the modified exendin-4 analog (with theoretical molecular weight of 44,300 Da) determined by SDS-PAGE was slightly higher than 43,000 Da (Figure 4), indicating that the product obtained was really the single mPEG derivatives (Mn: 40,000 Da) modified exendin-4 analog.

The collected eluent containing single mPEG modified exendin-4 analog was concentrated using ultrafiltration equipment (an ultrafiltration centrifuge tube or an ultrafiltration device). The concentrated solution was loaded onto a desalting column to exchange into acetic acid buffer. Finally, isotonic adjustment agent (such as mannitol and 0.9% NaCl) and 0.001 -1.0% (w/v) antibacterial reagent (such as 3-methylphenol) were added into the acetic acid buffer containing the modified exendin-4 analog.

### Example 6. 24-hour drug effect test of single mPEG derivatives (Mf: 5,000 Da and Mn: 21,000 Da) modified exendin analogs

Test Animal: C57 mice, 20g+/-2g, 8 mice for each group, a total of 4 groups.

Control Group 1: Sterile water was injected subcutaneously to each mouse.

Control Group 2: 0.025µg exendin-4 analog was injected subcutaneously to each mouse.

Drug Group 1: 0.625µg of exendin-4 analog modified by single mPEG derivatives (Mf: 5,000 Da) was injected subcutaneously to each mouse.

Drug Group 2: 0.625µg of exendin-4 analog modified by single mPEG derivatives (Mn: 21,000 Da) was injected subcutaneously to each mouse.

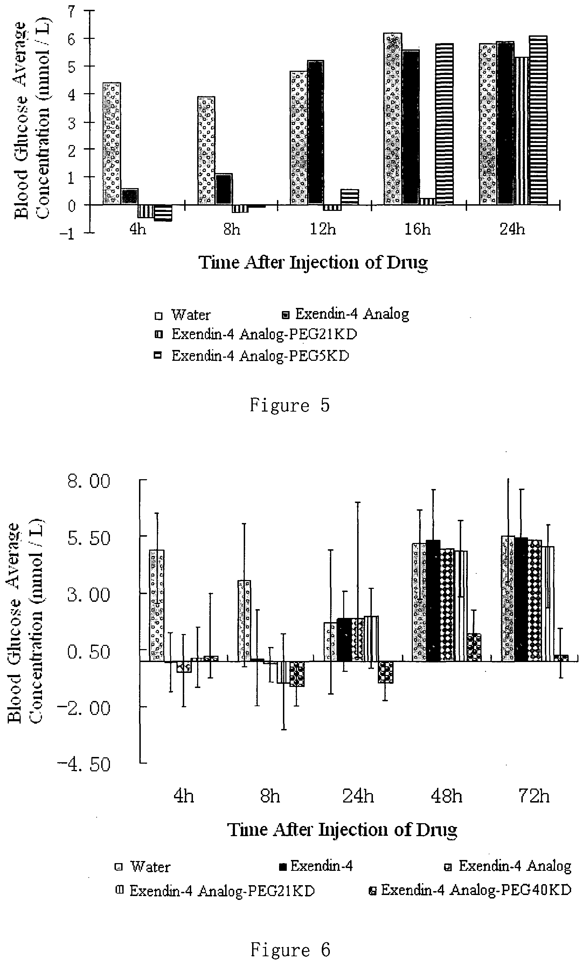

Blood sample was taken from each mice group at 4, 8, 12, 16 and 24 hours after drug injection. 200µl of 20% glucose solution was injected into the abdomen of each mouse 30 minutes before the blood sampling. The blood sugar level was measured using a glucose reagent kit (Shanghai ShenSuo Reagents Co., Ltd.). As shown in Figure 5, the exendin-4 analogs modified with mPEG derivatives (Mn: 21,000 Da) remained effective 16 hours after injection. The effective period for exendin-4 analog modified with mPEG (Mf: 5,000 Da) was between 12 to 16 hours after drug injection. The effective period for un-modified exendin-4 analogs was less than 12 hours. The effective period for lowering the blood sugar level was longer using exendin-4 analogs modified by linear mPEG derivatives with a larger molecular weight than exendin-4 analogs modified by linear mPEG derivatives with a smaller molecular weight.

### Example 7. 72-hour drug effect test of exendin analogs modified with linear mPEG (Mn=21,000 Da) and branched mPEG having the structure of Formula IV (Mn=40,000 Da)

Test Animal: C57 mice, 20 g +/- 2 g, 8 mice for each group, a total of 5 groups.

Control Group 1: Sterile water was injected subcutaneously to each mouse.

Control Group 2: 0.025µg of exendin-4 peptide was injected subcutaneously to each mouse.

Control Group 3: 0.025µg of exendin analog was injected subcutaneously to each mouse.

Drug Group 1: 0.625µg of exendin-4 analog modified by mPEG (Mn: 21,000 Da) was injected subcutaneously to each mouse.

Drug Group 2: 0.625µg of exendin-4 analog modified by mPEG (Mn: 40,000 Da) was injected subcutaneously to each mouse.

Blood samples were taken from each mice group at 4, 8, 24, 48 and 72 hours after drug injection. 200µl of 20% glucose solution was injected into the abdomen of each mouse 30 minutes before each blood sampling. The blood sugar level was measured using the glucose reagent kit. As shown in Figure 6, the exendin-4 analog modified by a branched PEG derivatives (Mn: 40,000 Da) remained effective for at least 72 hours after drug injection. The exendin-4 analog modified by linear mPEG derivatives (Mn: 21,000 Da) remained effective for about 24 hours after drug injection.

### Example 8. Drug effect test of Exendin-4 analogs modified by single mPEG derivatives

Test Samples: a total of 8 samples, exendin-4 analogs modified by single mPEG derivatives (Formula II) with molecular weights of 21,000 Da, 30,000 Da, 40,000 Da, respectively; exendin-4 analogs modified by single mPEG derivatives (Formula IV) with molecular weights of 21,000 Da, 30,000 Da, 40,000 Da, respectively; exendin-4 analogs modified by linear mPEG derivatives with molecular weights of 21,000 Da and 30,000 Da, respectively.

Test Animal: C57 mice, 20 g +/- 2 g, 3 sampling groups, 24 mice for each sampling group.

Because the mice could not endure frequent blood sampling, we designed to take blood from the sampling groups at different time after injecting the samples. The blood sampling schedule after drug injection was listed as follows:

| The First Group | The Second Group | The Third Group |
|---|---|---|
| 2, 8, 72 Hour | 4, 24, 48 Hour | 16, 36, 60 Hour |

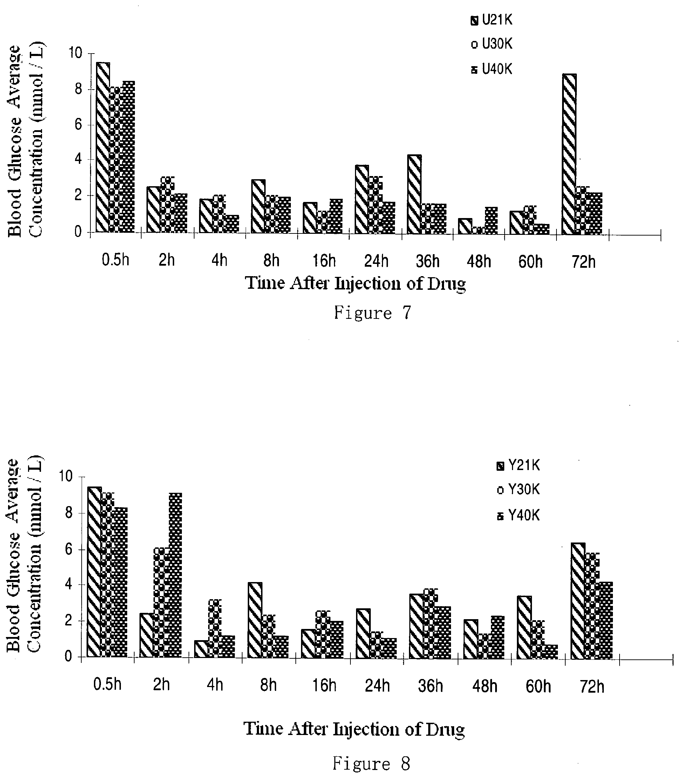

Each sampling group was further divided into 8 sub-groups. Every sub-group consisted of 3 mice. At the beginning of the test, each sub-group was injected with a different modified exendin-4 analog, which was referred to as L21K, L30K (modification by single linear mPEG derivatives having molecular weights of 21,000 Da and 30,000 Da, respectively); Y21K, Y30K, Y40K (modification by single mPEG derivatives having the structure of Formula IV and molecular weights of 21,000 Da, 30,000 Da and 40,000 Da, respectively), U21K, U30K, U40K (exendin-4 analogs modified with a single mPEG having the structure of Formula II and molecular weights of 21,000 Da, 30,000 Da and 40,000 Da, respectively). All of the different mPEG modified Exendin-4 analogs contained 0.625 µg of Exendin-4 analogs. 200µl 20% glucose was injected into the abdominal space of the mice right after the drug injection. After half an hour, blood samples were taken from all mice. Subsequent blood sampling was conducted according to the sampling schedule table above. 200µl 20% glucose was injected into the abdominal space of the mice half an hour prior to each blood sampling. Figures 7-9 showed that the molecular structure and weight of the PEG derivatives had great influence on the bioactivity of the exendin-4 analogs. The bioactivity of exendin-4 analogs modified by single mPEG having the structure of Formula II remains effective for a longer time than the exendin-4 analogs modified by PEG having the structure of Formula IV and the linear PEG in the 72 hours after drug injection. For the exendin-4 analogs modified by single PEG having the same branched structure, the greater molecular weight they had, the better bioactivity of lowering the blood sugar level they exhibited during the 72 hours. L30K has no noticeable bioactivity by the 72 hours after drug injection, which showed that higher molecular weight of linear PEG had a negative effect on the modified Exendin-4 analog's bioactivity.

### Example 9. Pharmacokinetics test of Exendin-4 analogs modified by single PEG

Test Animal: SD mice, male, 250g-300g, 2 groups, 4 mice for each group.

Drug Group 1: The mice were each injected with exendin-4 analogs (SEQ ID NO: 251) modified by single mPEG derivatives (Formula II, Mn: 30,000 Da) containing 4.375 µg Exendin-4 analogs (U30K).

Drug Group 2: The mice were each injected with exendin-4 analog (SEQ ID NO: 251) modified by single mPEG derivatives (Formula IV, Mn: 40,000 Da) containing 4.375 µg Exendin-4 analogs (Y40K).

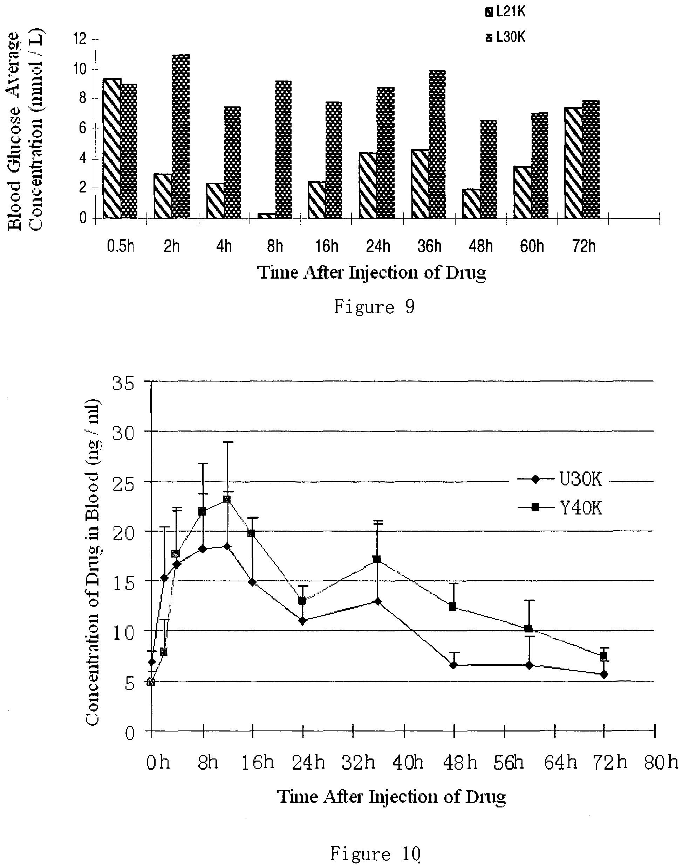

Blood samples were taken from every mice group at 0, 2, 4, 8, 12, 16, 24, 36, 48, 60 and 72 hours after the drug injection. The concentration of exendin-4 in the blood was tested using Enzyme Immunoassay kit (EIA) purchased from Phoenix Pharmaceuticals, Inc., California, U.S.A. The data was analyzed by Pharmaceutical Kinetics Software 1.0.2 (Shanghai Hongneng Software Co. Ltd.), and the results were shown in Figure 10. The time taken for half of the U30K to be absorbed was 10.78 hours and the time taken for half of the U30K to be eliminated was 21.44 hours. The time taken for half of the Y40K to be absorbed was 5.53 hours and the time taken for half of the Y40K to be eliminated was 40.77 hours. The time for the two samples to reach peak concentration in the blood was about 12 hours after injecting the drug. The peak concentration of the two samples in the blood was about 20ng/ml.

## Claims

1. A PEG modified exendin or exendin analog comprising one or more PEG derivatives linked to one or more amino acids of said exendin or exendin analog, wherein said one or more PEG derivatives have a molecular weight in the range of exceeding 20,000 Da to 50,000 Da and wherein said one or more PEG derivatives comprise the branched structure set forth in Formula I below: 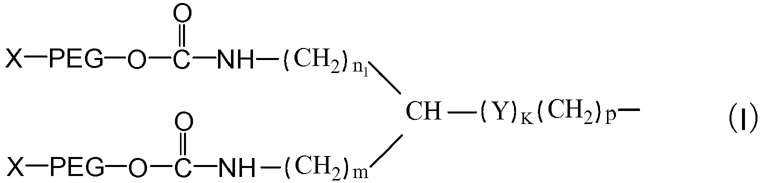 wherein X is hydrogen or a protecting group; PEG is -O(CH₂CH₂O)_{q-}, q is a positive integer; n₁ is an integer from 0-5; m is an integer from 0-5; Y is O, S, SO, SO₂ or NR₁, wherein R₁ is hydrogen or substituted or unsubstituted (C₁C₈) alkyl group or substituted or unsubstituted cycloalkyl group; k is 0 or 1 ; and p is an integer from 0-6.

2. A PEG modified exendin or exendin analog as claimed in claim 1, wherein said one or more PEG derivatives comprise the branched structure set forth in Formula II below: 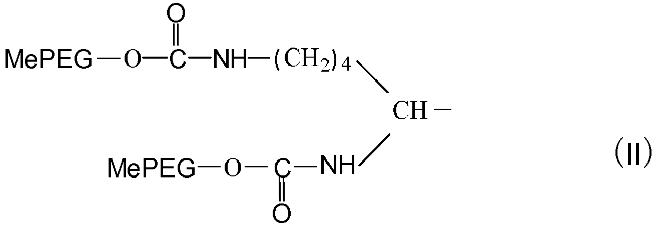 wherein PEG is -O(CH₂CH₂O)_{q-}, q is a positive integer, and Me is a methyl group.

3. A PEG modified exendin or exendin analog comprising one or more PEG derivatives linked to one or more amino acids of said exendin or exendin analog, wherein said one or more PEG derivatives have a molecular weight in the range of exceeding 20,000 Da to 50,000 Da and wherein said one or more PEG derivatives comprise the branched structure set forth in Formula III below: 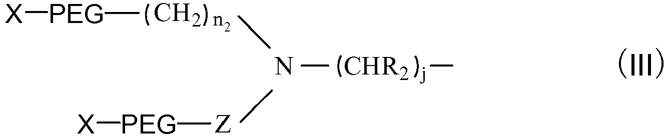 wherein X is hydrogen or a protecting group; PEG is -O(CH₂CH₂O)_{q-}, q is a positive integer; n₂ is an integer from 0-10; Z is a group selected from: (CH₂)ᵢ, (CH₂)ᵢOCO, (CH₂)NHCO, or (CH₂)ᵢCO, wherein i is an integer from 0-10; R₂ is hydrogen, substituted or unsubstituted (C₁-C₁₂) alkyl group, substituted aryl, aralkyl or heteroalkyl group; and j is an integer from 1-12.

4. A PEG modified exendin or exendin analog as claimed in claim 3, wherein said one or more PEG derivatives comprise the branched structure set forth in Formula IV below: 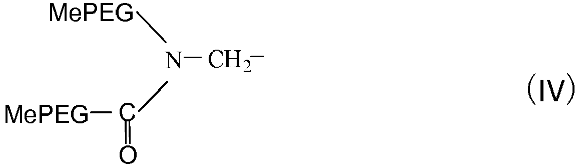 wherein PEG is -O(CH₂CH₂O)_{q-}, q is a positive integer, and Me is a methyl group.

5. A PEG modified exendin or exendin analog as claimed in any preceding claim, wherein said one or more PEG derivatives are activated to link to said exendin or exendin analog by N-Hydroxysuccinimide.

6. A PEG modified exendin or exendin analog as claimed in any preceding claim comprising one, two, three or four branched PEG derivatives.

7. A PEG modified exendin or exendin analog as claimed in claim 6 comprising one branched PEG derivative.

8. A PEG modified exendin or exendin analog as claimed in any preceding claim, wherein the exendin or exendin analog comprises the amino acid sequence set forth in SEQ ID NO: 1.

9. A PEG modified exendin or exendin analog as claimed in any of claims 1 to 7, wherein the exendin or exendin analog comprises the amino acid sequence set forth in SEQ ID NO: 2.

10. A PEG modified exendin or exendin analog as claimed in any of claims 1 to 7, wherein the exendin or exendin analog comprises one or more amino acid sequences set forth in SEQ ID NO: 3 to NO: 229.

11. A PEG modified exendin or exendin analog as claimed in any of claims 1 to 7, wherein the exendin or exendin analog comprises one or more amino acid sequences set forth in SEQ ID NO: 230 to NO: 265.

12. A composition comprising the PEG modified exendins or exendin analogs of any of claims 1 to 11.

13. Use of a PEG modified exendin or exendin analog of any of claims 1 to 11 or a composition of claim 12 in the manufacture of a medicament for preventing or treating diabetes mellitus.

14. A PEG modified exendin or exendin analog of any of claims 1 to 11 for use in treatment of diabetes mellitus.

## Patentansprüche

1. PEG-modifiziertes Exendin oder Exendin-Analogon, umfassend ein oder mehrere PEG-Derivate, die an eine oder mehrere Aminosäuren des Exendins oder Exendin-Analogons gebunden sind, wobei das eine oder die mehreren PEG-Derivate ein Molekulargewicht im Bereich von mehr als 20 000 Da bis 50 000 Da aufweisen und wobei das eine oder die mehreren PEG-Derivate die in nachstehender Formel I dargestellte verzweigte Struktur umfassen: 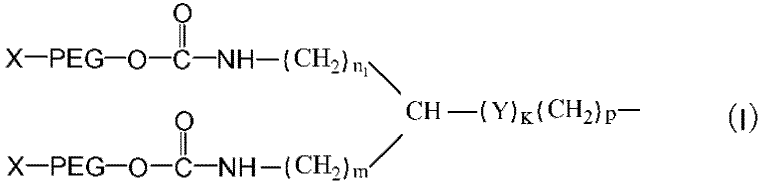 wobei X Wasserstoff oder eine Schutzgruppe ist; PEG -O(CH₂CH₂O)_{q}- ist, q eine positive ganze Zahl ist; n₁ eine ganze Zahl von 0-5 ist; m eine ganze Zahl von 0-5 ist; Y O, S, SO, SO₂ oder NR₁ ist,
wobei R₁ Wasserstoff oder ein substituierter oder unsubstituierter (C₁-C₈)-Alkylrest oder ein substituierter oder unsubstituierter Cycloalkylrest ist; k 0 oder 1 ist; und p eine ganze Zahl von 0-6 ist.

2. PEG-modifiziertes Exendin oder Exendin-Analogon nach Anspruch 1, wobei das eine oder die mehreren PEG-Derivate die in nachstehender Formel II dargestellte verzweigte Struktur umfassen: 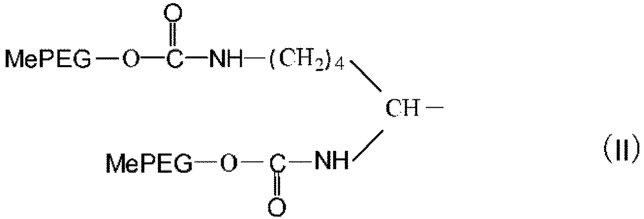 wobei PEG -O(CH₂CH₂O)_{q}- ist, q eine positive ganze Zahl ist, und Me eine Methylgruppe ist.

3. PEG-modifiziertes Exendin oder Exendin-Analogon, umfassend ein oder mehrere PEG-Derivate, die an eine oder mehrere Aminosäuren des Exendins oder Exendin-Analogons gebunden sind, wobei das eine oder die mehreren PEG-Derivate ein Molekulargewicht im Bereich von mehr als 20 000 Da bis 50 000 Da aufweisen und wobei das eine oder die mehreren PEG-Derivate die in nachstehender Formel III dargestellte verzweigte Struktur umfassen: 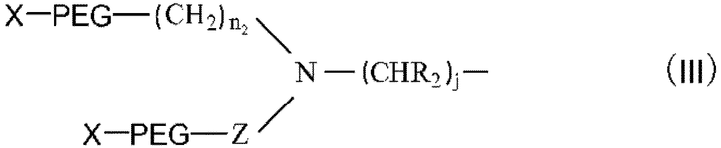 wobei X Wasserstoff oder eine Schutzgruppe ist; PEG -O(CH₂CH₂O)_{q}- ist, q eine positive ganze Zahl ist; n₂ eine ganze Zahl von 0-10 ist; Z eine Gruppe ist, ausgewählt aus (CH₂)ᵢ, (CH₂)ᵢOCO, (CH₂)ᵢNHCO oder (CH₂)CO, wobei i eine ganze Zahl von 0-10 ist; R₂ Wasserstoff, ein substituierter oder unsubstituierter (C₁-C₁₂)-Alkylrest, ein substituierter Aryl-, Aralkyl- oder Heteroalkylrest ist; und j eine ganze Zahl von 1-12 ist.

4. PEG-modifiziertes Exendin oder Exendin-Analogon nach Anspruch 3, wobei das eine oder die mehreren PEG-Derivate die in nachstehender Formel IV dargestellte verzweigte Struktur umfassen: 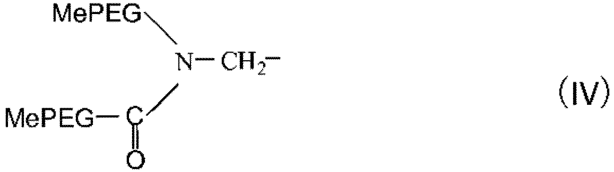 wobei PEG -O(CH₂CH₂O)_{q}- ist, q eine positive ganze Zahl ist, und Me eine Methylgruppe ist.

5. PEG-modifiziertes Exendin oder Exendin-Analogon nach einem vorangehenden Anspruch, wobei das eine oder die mehreren PEG-Derivate mit N-Hydroxysuccinimid aktiviert werden, um sie an das Exendin oder Exendin-Analogon zu binden.

6. PEG-modifiziertes Exendin oder Exendin-Analogon nach einem vorangehenden Anspruch, umfassend ein, zwei, drei oder vier verzweigte PEG-Derivate.

7. PEG-modifiziertes Exendin oder Exendin-Analogon nach Anspruch 6, umfassend ein verzweigtes PEG-Derivat.

8. PEG-modifiziertes Exendin oder Exendin-Analogon nach einem vorangehenden Anspruch, wobei das Exendin oder Exendin-Analogon die in SEQ ID Nr. 1 dargestellte Aminosäuresequenz umfasst.

9. PEG-modifiziertes Exendin oder Exendin-Analogon nach einem der Ansprüche 1 bis 7, wobei das Exendin oder Exendin-Analogon die in SEQ ID Nr. 2 dargestellte Aminosäuresequenz umfasst.

10. PEG-modifiziertes Exendin oder Exendin-Analogon nach einem der Ansprüche 1 bis 7, wobei das Exendin oder Exendin-Analogon eine oder mehrere Aminosäuresequenzen, dargestellt in SEQ ID Nr. 3 bis Nr. 229, umfasst.

11. PEG-modifiziertes Exendin oder Exendin-Analogon nach einem der Ansprüche 1 bis 7, wobei das Exendin oder Exendin-Analogon eine oder mehrere Aminosäuresequenzen, dargestellt in SEQ ID Nr. 230 bis Nr. 265, umfasst.

12. Zusammensetzung, umfassend die PEG-modifizierten Exendine oder Exendin-Analoga nach einem der Ansprüche 1 bis 11.

13. Verwendung eines PEG-modifizierten Exendins oder Exendin-Analogons nach einem der Ansprüche 1 bis 11 oder einer Zusammensetzung nach Anspruch 12 bei der Herstellung eines Medikaments zur Vorbeugung oder Behandlung von Diabetes mellitus.

14. PEG-modifiziertes Exendin oder Exendin-Analogon nach einem der Ansprüche 1 bis 11 zur Verwendung bei der Behandlung von Diabetes mellitus.

## Revendications

1. Exendine ou analogue d'exendine modifié(e) par PEG comprenant un ou plusieurs dérivés de PEG liés à un ou à plusieurs acides aminés de ladite exendine ou dudit analogue d'exendine, le ou les dérivés de PEG ayant un poids moléculaire compris entre 20.000 Da et 50.000 Da et le ou les dérivés de PEG comprenant la structure ramifiée décrite par la formule I suivante : 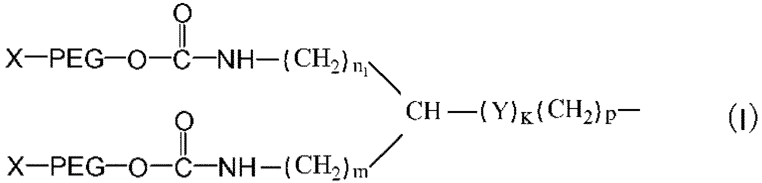 où X est de l'hydrogène ou un groupe protecteur ; PEG est -O(CH₂CH₂O)_{q}-, q est un entier positif ; n₁ est un entier entre 0 et 5 ; m est un entier entre 0 et 5 ; Y est O, S, SO, SO₂ ou NR₁, R₁ étant de l'hydrogène ou un groupe alkyle (C₁-C₈) substitué ou non substitué ou un groupe cycloalkyle substitué ou non substitué ; k est le nombre 0 ou le nombre 1 ; et p un entier entre 0 et 6.

2. Exendine ou analogue d'exendine modifié(e) par PEG selon la revendication 1, où le ou les dérivés de PEG comprennent la structure ramifiée décrite par la formule II suivante : 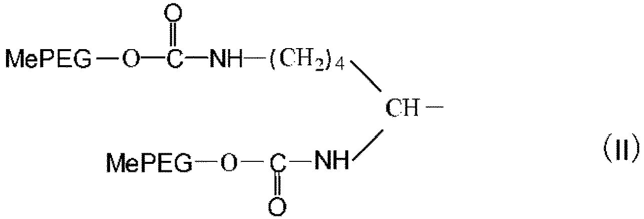 où PEG est -O(CH₂CH₂O)_{q}-, q un entier positif, et Me un groupe méthyle.

3. Exendine ou analogue d'exendine modifié(e) par PEG comprenant un ou plusieurs dérivés de PEG liés à un ou à plusieurs acides aminés de ladite exendine ou dudit analogue d'exendine, le ou les dérivés de PEG ayant un poids moléculaire compris entre 20.000 Da et 50.000 Da et le ou les dérivés de PEG comprenant la structure ramifiée décrite par la formule III suivante : 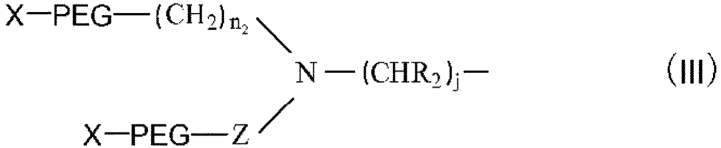 où X est de l'hydrogène ou un groupe protecteur ; PEG est -O(CH₂CH₂O)_{q}-, q est un entier positif ; n₂ est un entier entre 0 et 10 ; Z est un groupe sélectionné entre : (CH₂)ᵢ, (CH₂)₁OCO, (CH₂)₁NHCO, ou (CH₂)₁CO, i étant un entier entre 0 et 10 ; R₂ étant de l'hydrogène, un groupe alkyle (C₁-C₁₂) substitué ou non substitué, un groupe aryle, aralkyle ou hétéroalkyle substitué ou non substitué ; et j un entier entre 1 et 12.

4. Exendine ou analogue d'exendine modifié(e) par PEG selon la revendication 3, où le ou les dérivés de PEG comprennent la structure ramifiée décrite par la formule IV suivante : 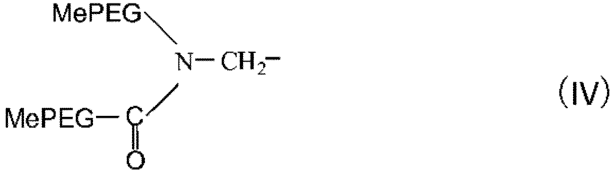 où PEG est -O(CH₂CH₂O)_{q}-, q un entier positif, et Me un groupe méthyle.

5. Exendine ou analogue d'exendine modifié(e) par PEG selon l'une des revendications précédentes, où le ou les dérivés de PEG sont activés pour lier l'exendine ou l'analogue d'exendine par N-hydroxysuccinimide.

6. Exendine ou analogue d'exendine modifié(e) par PEG selon l'une des revendications précédentes, comprenant un, deux, trois ou quatre dérivés PEG ramifiés.

7. Exendine ou analogue d'exendine modifié(e) par PEG selon la revendication 6, comprenant un dérivé PEG ramifié.

8. Exendine ou analogue d'exendine modifié(e) par PEG selon l'une des revendications précédentes, où l'exendine ou l'analogue d'exendine comprend la séquence d'acides aminés décrite en SEQ ID NO: 1.

9. Exendine ou analogue d'exendine modifié(e) par PEG selon l'une des revendications 1 à 7, où l'exendine ou l'analogue d'exendine comprend la séquence d'acides aminés décrite en SEQ ID NO: 2.

10. Exendine ou analogue d'exendine modifié(e) par PEG selon l'une des revendications 1 à 7, où l'exendine ou l'analogue d'exendine comprend une ou plusieurs séquences d'acides aminés décrites en SEQ ID NO: 3 à NO: 229.

11. Exendine ou analogue d'exendine modifié(e) par PEG selon l'une des revendications 1 à 7, où l'exendine ou l'analogue d'exendine comprend une ou plusieurs séquences d'acides aminés décrites en SEQ ID NO: 230 to NO: 265.

12. Composition comprenant l'exendine ou l'analogue d'exendine modifié(e) par PEG selon l'une des revendications 1 à 11.

13. Utilisation d'exendine ou d'analogue d'exendine modifié(e) par PEG selon l'une des revendications 1 à 11 ou d'une composition selon la revendication 12 pour la production d'un médicament de prévention ou de traitement du diabète sucré.

14. Exendine ou analogue d'exendine modifié(e) par PEG selon l'une des revendications 1 à 11 utilisable pour le traitement du diabète sucré.


### SEQUENCE LISTING

<110> Shanghai Huayi Bio-lab Co., Ltd.
   <120> PEG MODIFIED EXENDIN OR EXENDIN ANALOG AND COMPOSITIONS AND USE THEREOF
   <130> 57783.8008.WOOO
   <160> 269
   <170> PatentIn version 3.3
<210> 1
   <211> 39
   <212> PRT
   <213> Exendin-3
<400> 1
   His Ser Asp Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 2
   <211> 39
   <212> PRT
   <213> Exendin-4
<400> 2
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 3
   <211> 30
   <212> PRT
   <213> Exendin Analog 1
<400> 3
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly
   20 25 30
<210> 4
   <211> 30
   <212> PRT
   <213> Exendin Analog 2
<400> 4
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly
   20 25 30
<210> 5
   <211> 28
   <212> PRT
   <213> Exendin Analog 3
<400> 5
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 6
   <211> 39
   <212> PRT
   <213> Exendin Analog 4
<400> 6
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro Ser
   35
<210> 7
   <211> 28
   <212> PRT
   <213> Exendin Analog 5
<400> 7
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 8
   <211> 28
   <212> PRT
   <213> Exendin Analog 6
<400> 8
   His Gly Glu Gly Thr Phe Thr Ser Asp Leu Ser Lys Gln Leu Glu Glu
   1 5 10 15
   Glu Ala Val Arg Leu Ala Ile Glu Phe Leu Lys Asn
   20 25
<210> 9
   <211> 18
   <212> PRT
   <213> Exendin Analog 7
<220>
   <221> misc_feature
   <222> (9)..(9)
   <223> Xaa stands for Pgly
<400> 9
   His Gly Glu Phe Thr Ser Asp Leu Xaa Phe Ile Glu Phe Pro Pro Pro
   1 5 10 15
   Pro Ser
<210> 10
   <211> 18
   <212> PRT
   <213> Exendin Analog 8
<220>
   <221> misc_feature
   <222> (10).. (10)
   <223> Xaa stands for naph
<400> 10
   His Gly Glu Phe Thr Ser Asp Leu Met Xaa Ile Glu Trp Pro Pro Pro
   1 5 10 15
   Pro Ser
<210> 11
   <211> 20
   <212> PRT
   <213> Exendin Analog 9
<400> 11
   His Gly Glu Phe Thr Ser Asp Leu Met Phe Val Ala Leu Glu Trp Pro
   1 5 10 15
   Pro Pro Pro Ser
   20
<210> 12
   <211> 20
   <212> PRT
   <213> Exendin Analog 10
<400> 12
   His Gly Glu Phe Thr Ser Asp Leu Leu Phe Val Ala Leu Glu Phe Pro
   1 5 10 15
   Pro Pro Pro Ser
   20
<210> 13
   <211> 18
   <212> PRT
   <213> Exendin Analog 11
<220>
   <221> misc_feature
   <222> (11)..(11)
   <223> Xaa stands for tBug
<400> 13
   His Gly Glu Phe Thr Ser Asp Leu Met Phe Xaa Glu Trp Pro Pro Pro
   1 5 10 15
   Pro Ser
<210> 14
   <211> 18
   <212> PRT
   <213> Exendin Analog 12
<220>
   <221> misc_feature
   <222> (11).. (11)
   <223> Xaa stands for tBug
<400> 14
   His Gly Glu Phe Thr Ser Asp Leu Leu Phe Xaa Glu Phe Pro Pro Pro
   1 5 10 15
   Pro Ser
<210> 15
   <211> 18
   <212> PRT
   <213> Exendin Analog 13
<400> 15
   His Gly Glu Phe Thr Ser Asp Leu Met Phe Ile Asp Trp Pro Pro Pro
   1 5 10 15
   Pro Ser
<210> 16
   <211> 18
   <212> PRT
   <213> Exendin Analog 14
<400> 16
   His Ala Glu Phe Thr Ser Asp Leu Leu Phe Ile Glu Phe Pro Pro Pro
   1 5 10 15
   Pro Ser
<210> 17
   <211> 18
   <212> PRT
   <213> Exendin Analog 15
<220>
   <221> misc_feature
   <222> (14)..(17)
   <223> Xaa stands for hPro
<400> 17
   His Gly Glu Phe Thr Ser Asp Leu Met Phe Ile Glu Trp Xaa Xaa Xaa
   1 5 10 15
   Xaa Ser
<210> 18
   <211> 18
   <212> PRT
   <213> Exendin Analog 16
<220>
   <221> misc_feature
   <222> (15)..(17)
   <223> Xaa stands for hPro
<400> 18
   His Gly Glu Phe Thr Ser Asp Leu Met Phe Ile Glu Trp Pro Xaa Xaa
   1 5 10 15
   Xaa Ser
<210> 19
   <211> 17
   <212> PRT
   <213> Exendin Analog 17
<220>
   <221> misc_feature
   <222> (14)..(16)
   <223> Xaa stands for hPro
<400> 19
   His Gly Glu Phe Thr Ser Asp Leu Met Phe Ile Glu Trp Xaa Xaa Xaa
   1 5 10 15
   Ser
<210> 20
   <211> 18
   <212> PRT
   <213> Exendin Analog 18
<220>
   <221> misc _feature
   <222> (15)..(17)
   <223> Xaa stands for hPro
<400> 20
   His Gly Glu Phe Thr Ser Asp Leu Met Phe Ile Glu Trp Pro Xaa Xaa
   1 5 10 15
   Xaa Ser
<210> 21
   <211> 18
   <212> PRT
   <213> Exendin Analog 19
<220>
   <221> mise_feature
   <222> (14)..(17)
   <223> Xaa stands for hPro
<400> 21
   His Gly Glu Phe Thr Ser Asp Leu Leu Phe Ile Glu Phe Xaa Xaa Xaa
   1 5 10 15
   Xaa Ser
<210> 22
   <211> 18
   <212> PRT
   <213> Exendin Analog 20
<220>
   <221> misc_feature
   <222> (14)..(17)
   <223> Xaa stands for hPro
<400> 22
   His Gly Glu Phe Thr Ser Asp Leu Leu Phe Ile Glu Phe Xaa Xaa Xaa
   1 5 10 15
   Xaa Ser
<210> 23
   <211> 18
   <212> PRT
   <213> Exendin Analog 21
<220>
   <221> misc_feature
   <222> (14)..(17)
   <223> Xaa stands for MeAla
<400> 23
   His Gly Glu Phe Thr Ser Asp Leu Met Phe Ile Glu Trp Xaa Xaa Xaa
   1 5 10 15
   Xaa Ser
<210> 24
   <211> 18
   <212> PRT
   <213> Exendin Analog 22
<220>
   <221> misc_feature
   <222> (15).. (17)
   <223> Xaa stands for MeAla
<400> 24
   His Gly Glu Phe Thr Ser Asp Leu Met Phe Ile Glu Trp Pro Xaa Xaa
   1 5 10 15
   Xaa Ser
<210> 25
   <211> 18
   <212> PRT
   <213> Exendin Analog 23
<220>
   <221> misc_feature
   <222> (14)..(17)
   <223> Xaa stands for MeAla
<400> 25
   His Gly Glu Phe Thr Ser Asp Leu Leu Phe Ile Glu Phe Xaa Xaa Xaa
   1 5 10 15
   Xaa Ser
<210> 26
   <211> 30
   <212> PRT
   <213> Exendin Analog 24
<400> 26
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly
   20 25 30
<210> 27
   <211> 28
   <212> PRT
   <213> Exendin Analog 25
<400> 27
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn
               20                  25
<210> 28
   <211> 28
   <212> PRT
   <213> Exendin Analog 26
<400> 28
   His Gly Glu Gly Thr Phe Thr Ser Asp Leu Ser Lys Gln Leu Glu Glu
   1               5                   10                  15
   Glu Ala Val Arg Leu Phe Ile Glu Phe Leu Lys Asn
               20                  25
<210> 29
   <211> 28
   <212> PRT
   <213> Exendin Analog 27
<400> 29
   His Ala Glu Gly Thr Phe Thr Ser Asp Leu Ser Lys Gln Leu Glu Glu
   1               5                   10                  15
   Glu Ala Val Arg Leu Phe Ile Glu Phe Leu Lys Asn
               20                  25
<210> 30
   <211> 28
   <212> PRT
   <213> Exendin Analog 28
<400> 30
   His Gly Glu Gly Ala Phe Thr Ser Asp Leu Ser Lys Gln Leu Glu Glu
   1               5                   10                  15
   Glu Ala Val Arg Leu Phe Ile Glu Phe Leu Lys Asn
               20                  25
<210> 31
   <211> 28
   <212> PRT
   <213> Exendin Analog 29
<400> 31
   His Gly Glu Gly Thr Ala Thr Ser Asp Leu Ser Lys Gln Leu Glu Glu
   1 5 10 15
   Glu Ala Val Arg Leu Phe Ile Glu Phe Leu Lys Asn
   20 25
<210> 32
   <211> 28
   <212> PRT
   <213> Exendin Analog 30
<400> 32
   His Gly Glu Gly Thr Phe Thr Ala Asp Leu Ser Lys Gln Leu Glu Glu
   1 5 10 15
   Glu Ala Val Arg Leu Phe Ile Glu Phe Leu Lys Asn
   20 25
<210> 33
   <211> 28
   <212> PRT
   <213> Exendin Analog 31
<400> 33
   His Gly Glu Gly Thr Phe Thr Ser Asp Ala Ser Lys Gln Leu Glu Glu
   1 5 10 15
   Glu Ala Val Arg Leu Phe Ile Glu Phe Leu Lys Asn
   20 25
<210> 34
   <211> 28
   <212> PRT
   <213> Exendin Analog 32
<400> 34
   His Gly Glu Gly Thr Phe Thr Ser Asp Leu Ala Lys Gln Leu Glu Glu
   1 5 10 15
   Glu Ala Val Arg Leu Phe Ile Glu Phe Leu Lys Asn
   20 25
<210> 35
   <211> 28
   <212> PRT
   <213> Exendin Analog 33
<400> 35
   His Gly Glu Gly Thr Phe Thr Ser Asp Leu Ser Ala Gln Leu Glu Glu
   1 5 10 15
   Glu Ala Val Arg Leu Phe Ile Glu Phe Leu Lys Asn
   20 25
<210> 36
   <211> 28
   <212> PRT
   <213> Exendin Analog 34
<400> 36
   His Gly Glu Gly Thr Phe Thr Ser Asp Leu Ser Lys Ala Leu Glu Glu
   1 5 10 15
   Glu Ala Val Arg Leu Phe Ile Glu Phe Leu Lys Asn
   20 25
<210> 37
   <211> 28
   <212> PRT
   <213> Exendin Analog 35
<400> 37
   His Gly Glu Gly Thr Phe Thr Ser Asp Leu Ser Lys Gln Ala Glu Glu
   1 5 10 15
   Glu Ala Val Arg Leu Phe Ile Glu Phe Leu Lys Asn
   20 25
<210> 38
   <211> 28
   <212> PRT
   <213> Exendin Analog 36
<400> 38
   His Gly Glu Gly Thr Phe Thr Ser Asp Leu Ser Lys Gln Leu Ala Glu
   1 5 10 15
   Glu Ala Val Arg Leu Phe Ile Glu Phe Leu Lys Asn
   20 25
<210> 39
   <211> 28
   <212> PRT
   <213> Exendin Analog 37
<400> 39
   His Gly Glu Gly Thr Phe Thr Ser Asp Leu Ser Lys Gln Leu Glu Ala
   1 5 10 15
   Glu Ala Val Arg Leu Phe Ile Glu Phe Leu Lys Asn
   20 25
<210> 40
   <211> 28
   <212> PRT
   <213> Exendin Analog 38
<400> 40
   His Gly Glu Gly Thr Phe Thr Ser Asp Leu Ser Lys Gln Leu Glu Glu
   1 5 10 15
   Ala Ala Val Arg Leu Phe Ile Glu Phe Leu Lys Asn
   20 25
<210> 41
   <211> 28
   <212> PRT
   <213> Exendin Analog 39
<400> 41
   His Gly Glu Gly Thr Phe Thr Ser Asp Leu Ser Lys Gln Leu Glu Glu
   1 5 10 15
   Glu Ala Ala Arg Leu Phe Ile Glu Phe Leu Lys Asn
   20 25
<210> 42
   <211> 28
   <212> PRT
   <213> Exendin Analog 40
<400> 42
   His Gly Glu Gly Thr Phe Thr Ser Asp Leu Ser Lys Gln Leu Glu Glu
   1 5 10 15
   Glu Ala Val Ala Leu Phe Ile Glu Phe Leu Lys Asn
   20 25
<210> 43
   <211> 28
   <212> PRT
   <213> Exendin Analog 41
<400> 43
   His Gly Glu Gly Thr Phe Thr Ser Asp Leu Ser Lys Gln Leu Glu Glu
   1 5 10 15
   Glu Ala Val Arg Ala Phe Ile Glu Phe Leu Lys Asn
   20 25
<210> 44
   <211> 28
   <212> PRT
   <213> Exendin Analog 42
<400> 44
   His Gly Glu Gly Thr Phe Thr Ser Asp Leu Ser Lys Gln Leu Glu Glu
   1               5                   10                  15
   Glu Ala Val Arg Leu Phe Ile Ala Phe Leu Lys Asn
               20                  25
<210> 45
   <211> 28
   <212> PRT
   <213> Exendin Analog 43
<400> 45
   His Gly Glu Gly Thr Phe Thr Ser Asp Leu Ser Lys Gln Leu Glu Glu
   1               5                   10                  15
   Glu Ala Val Arg Leu Phe Ile Glu Ala Leu Lys Asn
               20                  25
<210> 46
   <211> 28
   <212> PRT
   <213> Exendin Analog 44
<400> 46
   His Gly Glu Gly Thr Phe Thr Ser Asp Leu Ser Lys Gln Leu Glu Glu
   1               5                   10                  15
   Glu Ala Val Arg Leu Phe Ile Glu Phe Ala Lys Asn
               20                  25
<210> 47
   <211> 28
   <212> PRT
   <213> Exendin Analog 45
<400> 47
   His Gly Glu Gly Thr Phe Thr Ser Asp Leu Ser Lys Gln Leu Glu Glu
   1               5                   10                  15
   Glu Ala Val Arg Leu Phe Ile Glu Phe Leu Ala Asn
               20                  25
<210> 48
   <211> 28
   <212> PRT
   <213> Exendin Analog 46
<400> 48
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Ala
   20 25
<210> 49
   <211> 38
   <212> PRT
   <213> Exendin Analog 47
<400> 49
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro
   35
<210> 50
   <211> 38
   <212> PRT
   <213> Exendin Analog 48
<400> 50
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro
   35
<210> 51
   <211> 37
   <212> PRT
   <213> Exendin Analog 49
<400> 51
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro
   35
<210> 52
   <211> 37
   <212> PRT
   <213> Exendin Analog 50
<400> 52
   His Gly Glu Gly Thr Phe Thr Ser Asp Leu Ser Lys Gln Leu Glu Glu
   1               5                   10                  15
   Glu Ala Val Arg Leu Phe Ile Glu Phe Leu Lys Asn Gly Gly Pro Ser
               20                  25                  30
   Ser Gly Ala Pro Pro
           35
<210> 53
   <211> 36
   <212> PRT
   <213> Exendin Analog 51
<400> 53
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro
           35
<210> 54
   <211> 36
   <212> PRT
   <213> Exendin Analog 52
<400> 54
   His Gly Glu Gly Thr Phe Thr Ser Asp Leu Ser Lys Gln Leu Glu Glu
   1               5                   10                  15
   Glu Ala Val Arg Leu Phe Ile Glu Phe Leu Lys Asn Gly Gly Pro Ser
               20                  25                  30
   Ser Gly Ala Pro
           35
<210> 55
   <211> 35
   <212> PRT
   <213> Exendin Analog 53
<400> 55
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala
   35
<210> 56
   <211> 35
   <212> PRT
   <213> Exendin Analog 54
<400> 56
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala
   35
<210> 57
   <211> 34
   <212> PRT
   <213> Exendin Analog 55
<400> 57
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly
<210> 58
   <211> 34
   <212> PRT
   <213> Exendin Analog 56
<400> 58
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly
<210> 59
   <211> 33
   <212> PRT
   <213> Exendin Analog 57
<400> 59
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser
<210> 60
   <211> 33
   <212> PRT
   <213> Exendin Analog 58
<400> 60
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser
<210> 61
   <211> 32
   <212> PRT
   <213> Exendin Analog 59
<400> 61
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
<210> 62
   <211> 32
   <212> PRT
   <213> Exendin Analog 60
<400> 62
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
<210> 63
   <211> 31
   <212> PRT
   <213> Exendin Analog 61
<400> 63
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro
   20 25 30
<210> 64
   <211> 31
   <212> PRT
   <213> Exendin Analog 62
<400> 64
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro
   20 25 30
<210> 65
   <211> 30
   <212> PRT
   <213> Exendin Analog 63
<400> 65
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly
   20 25 30
<210> 66
   <211> 29
   <212> PRT
   <213> Exendin Analog 64
<400> 66
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly
   20 25
<210> 67
   <211> 29
   <212> PRT
   <213> Exendin Analog 65
<400> 67
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly
   20 25
<210> 68
   <211> 38
   <212> PRT
   <213> Exendin Analog 66
<220>
   <221> misc_feature
   <222> (31)..(31)
   <223> Xaa stands for hPro
<220>
   <221> misc_feature
   <222> (36)..(38)
   <223> Xaa stands for hPro
<400> 68
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Xaa Ser
   20 25 30
   Ser Gly Ala Xaa Xaa Xaa
   35
<210> 69
   <211> 38
   <212> PRT
   <213> Exendin Analog 67
<220>
   <221> misc_feature
   <222> (36)..(38)
   <223> Xaa stands for hPro
<400> 69
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Xaa Xaa Xaa
   35
<210> 70
   <211> 37
   <212> PRT
   <213> Exendin Analog 68
<220>
   <221> misc_feature
   <222> (31)..(31)
   <223> Xaa stands for Nme
<400> 70
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Xaa Ser
               20                  25                  30
   Ser Gly Ala Pro Pro
           35
<210> 71
   <211> 37
   <212> PRT
   <213> Exendin Analog 69
<220>
   <221> misc_feature
   <222> (31).. (31)
   <223> Xaa stands for Nme
<220>
   <221> misc_feature
   <222> (36)..(37)
   <223> Xaa stands for Nme
<400> 71
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Xaa Ser
               20                  25                  30
   Ser Gly Ala Xaa Xaa
           35
<210> 72
   <211> 37
   <212> PRT
   <213> Exendin Analog 70
<220>
   <221> misc_feature
   <222> (31)..(31)
   <223> Xaa stands for hPro
<220>
   <221> misc_feature
   <222> (36)..(37)
   <223> Xaa stands for hPro
<400> 72
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Xaa Ser
   20 25 30
   Ser Gly Ala Xaa Xaa
   35
<210> 73
   <211> 36
   <212> PRT
   <213> Exendin Analog 71
<220>
   <221> misc_feature
   <222> (31)..(31)
   <223> Xaa stands for hPro
<220>
   <221> misc_feature
   <222> (36)..(36)
   <223> Xaa stands for hPro
<400> 73
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Xaa Ser
   20 25 30
   Ser Gly Ala Xaa
   35
<210> 74
   <211> 35
   <212> PRT
   <213> Exendin Analog 72
<400> 74
   Arg Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala
   35
<210> 75
   <211> 30
   <212> PRT
   <213> Exendin Analog 73
<400> 75
   His Gly Asp Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly
               20                  25                  30
<210> 76
   <211> 28
   <212> PRT
   <213> Exendin Analog 74
<220>
   <221> misc_feature
   <222> (6)..(6)
   <223> Xaa stands for naph
<400> 76
   His Gly Glu Gly Thr Xaa Thr Ser Asp Leu Ser Lys Gln Leu Glu Glu
   1               5                   10                  15
   Glu Ala Val Arg Leu Phe Ile Glu Phe Leu Lys Asn
               20                  25
<210> 77
   <211> 28
   <212> PRT
   <213> Exendin Analog 75
<400> 77
   His Gly Glu Gly Thr Phe Ser Ser Asp Leu Ser Lys Gln Met Glu Glu
   1               5                   10                  15
   Glu Ala Val Arg Leu Phe Ile Glu Trp Leu Lys Asn
               20                  25
<210> 78
   <211> 28
   <212> PRT
   <213> Exendin Analog 76
<400> 78
   His Gly Glu Gly Thr Phe Ser Thr Asp Leu Ser Lys Gln Met Glu Glu
   1               5                   10                  15
   Glu Ala Val Arg Leu Phe Ile Glu Trp Leu Lys Asn
               20                  25
<210> 79
   <211> 28
   <212> PRT
   <213> Exendin Analog 77
<400> 79
   His Gly Glu Gly Thr Phe Thr Ser Glu Leu Ser Lys Gln Met Ala Glu
   1               5                   10                  15
   Glu Ala Val Arg Leu Phe Ile Glu Trp Leu Lys Asn
               20                  25
<210> 80
   <211> 28
   <212> PRT
   <213> Exendin Analog 78
<220>
   <221> misc_feature
   <222> (10)..(10)
   <223> Xaa stands for Pgly
<400> 80
   His Gly Glu Gly Thr Phe Thr Ser Asp Xaa Ser Lys Gln Leu Glu Glu
   1               5                   10                  15
   Glu Ala Val Arg Leu Phe Ile Glu Phe Leu Lys Asn
               20                  25
<210> 81
   <211> 28
   <212> PRT
   <213> Exendin Analog 79
<220>
   <221> misc_feature
   <222> (22)..(22)
   <223> Xaa stands for naph
<400> 81
   His Gly Glu Gly Thr Phe Thr Ser Asp Leu Ser Lys Gln Leu Glu Glu
   1               5                   10                  15
   Glu Ala Val Arg Leu Xaa Ile Glu Phe Leu Lys Asn
               20                  25
<210> 82
   <211> 28
   <212> PRT
   <213> Exendin Analog 80
<220>
   <221> misc_feature
   <222> (23)..(23)
   <223> Xaa stands for tBug
<400> 82
   His Gly Glu Gly Thr Phe Thr Ser Asp Leu Ser Lys Gln Met Glu Glu
   1               5                   10                  15
   Glu Ala Val Arg Leu Phe Xaa Glu Trp Leu Lys Asn
               20                  25
<210> 83
   <211> 28
   <212> PRT
   <213> Exendin Analog 81
<400> 83
   His Gly Glu Gly Thr Phe Thr Ser Asp Leu Ser Lys Gln Leu Glu Glu
   1               5                   10                  15
   Glu Ala Val Arg Leu Phe Ile Asp Phe Leu Lys Asn
               20                  25
<210> 84
   <211> 33
   <212> PRT
   <213> Exendin Analog 82
<400> 84
   His Gly Glu Gly Thr Phe Thr Ser Asp Ala Ser Lys Gln Leu Glu Glu
   1               5                   10                  15
   Glu Ala Val Arg Leu Phe Ile Glu Phe Leu Lys Asn Gly Gly Pro Ser
               20                  25                  30
   Ser
<210> 85
   <211> 29
   <212> PRT
   <213> Exendin Analog 83
<400> 85
   His Gly Glu Gly Thr Phe Thr Ser Asp Ala Ser Lys Gln Met Glu Glu
   1               5                   10                  15
   Glu Ala Val Arg Leu Phe Ile Glu Trp Leu Lys Asn Gly
               20                  25
<210> 86
   <211> 37
   <212> PRT
   <213> Exendin Analog 84
<220>
   <221> misc_feature
   <222> (31)..(31)
   <223> Xaa stands for Pgly
<220>
   <221> misc_feature
   <222> (36)..(37)
   <223> Xaa stands for Pgly
<400> 86
   His Gly Glu Gly Thr Phe Thr Ser Asp Ala Ser Lys Gln Met Glu Glu
   1               5                   10                  15
   Glu Ala Val Arg Leu Phe Ile Glu Trp Leu Lys Asn Gly Gly Xaa Ser
               20                  25                  30
   Ser Gly Ala Xaa Xaa
           35
<210> 87
   <211> 27
   <212> PRT
   <213> Exendin Analog 85
<220>
   <221> misc_feature
   <222> (1)..(1)
   <223> Xaa stands for 4-ImidazolylPpionyl-G
<220>
   <221> misc_feature
   <222> (26)..(26)
   <223> Xaa stands for 4-ImidazolylPpionyl-G
<400> 87
   Xaa Glu Gly Thr Phe Thr Ser Asp Leu Ser Lys Gln Met Glu Glu Glu
   1               5                   10                  15
   Ala Val Arg Leu Phe Ile Glu Trp Leu Xaa Asn
               20                  25
<210> 88
   <211> 27
   <212> PRT
   <213> Exendin Analog 86
<220>
   <221> misc_feature
   <222> (1)..(1)
   <223> Xaa stands for 4-ImidazolylPpionyl-G
<220>
   <221> misc_feature
   <222> (26)..(26)
   <223> Xaa stands for 4-ImidazolylPpionyl-G
<400> 88
   Xaa Glu Gly Thr Phe Thr Ser Asp Leu Ser Lys Gln Leu Glu Glu Glu
   1 5 10 15
   Ala Val Arg Leu Phe Ile Glu Phe Leu Xaa Asn
   20 25
<210> 89
   <211> 29
   <212> PRT
   <213> Exendin Analog 87
<220>
   <221> misc_feature
   <222> (1)..(1)
   <223> Xaa stands for 4-ImidazolylPpionyl-G
<220>
   <221> misc_feature
   <222> (26)..(26)
   <223> Xaa stands for 4-ImidazolylPpionyl-G
<400> 89
   Xaa Glu Gly Thr Phe Thr Ser Asp Leu Ser Lys Gln Met Glu Glu Glu
   1 5 10 15
   Ala Val Arg Leu Phe Ile Glu Trp Leu Xaa Asn Gly Gly
   20 25
<210> 90
   <211> 29
   <212> PRT
   <213> Exendin Analog 88
<220>
   <221> misc_feature
   <222> (1)..(1)
   <223> Xaa stands for 4-ImidazolylPpionyl-G
<220>
   <221> misc_feature
   <222> (26)..(26)
   <223> Xaa stands for 4-ImidazolylPpionyl-G
<400> 90
   Xaa Glu Gly Thr Phe Thr Ser Asp Leu Ser Lys Gln Leu Glu Glu Glu
   1 5 10 15
   Ala Val Arg Leu Phe Ile Glu Phe Leu Xaa Asn Gly Gly
   20 25
<210> 91
   <211> 27
   <212> PRT
   <213> Exendin Analog 89
<220>
   <221> misc_feature
   <222> (1)..(1)
   <223> Xaa stands for 4-ImidazolylPpionyl-G
<220>
   <221> misc_feature
   <222> (27)..(27)
   <223> Xaa stands for 4-ImidazolylPpionyl-G
<400> 91
   Xaa Glu Gly Thr Phe Thr Ser Asp Leu Ser Lys Gln Met Glu Glu Glu
   1               5                   10                  15
   Ala Val Arg Leu Phe Ile Glu Trp Leu Asn Xaa
               20                  25
<210> 92
   <211> 27
   <212> PRT
   <213> Exendin Analog 90
<220>
   <221> misc_feature
   <222> (1)..(1)
   <223> Xaa stands for 4-ImidazolylPpionyl-G
<220>
   <221> misc_feature
   <222> (27).. (27)
   <223> Xaa stands for 4-ImidazolylPpionyl-G
<400> 92
   Xaa Glu Gly Thr Phe Thr Ser Asp Leu Ser Lys Gln Leu Glu Glu Glu
   1               5                   10                  15
   Ala Val Arg Leu Phe Ile Glu Phe Leu Asn Xaa
               20                  25
<210> 93
   <211> 29
   <212> PRT
   <213> Exendin Analog 91
<220>
   <221> misc_feature
   <222> (1)..(1)
   <223> Xaa stands for 4-ImidazolylPpionyl-G
<220>
   <221> misc_feature
   <222> (27).. (27)
   <223> Xaa stands for 4-ImidazolylPpionyl-G
<400> 93
   Xaa Glu Gly Thr Phe Thr Ser Asp Leu Ser Lys Gln Met Glu Glu Glu
   1 5 10 15
   Ala Val Arg Leu Phe Ile Glu Trp Leu Asn Xaa Gly Gly
   20 25
<210> 94
   <211> 29
   <212> PRT
   <213> Exendin Analog 92
<220>
   <221> misc_feature
   <222> (1)..(1)
   <223> Xaa stands for 4-ImidazolylPpionyl-G
<220>
   <221> misc_feature
   <222> (27)..(27)
   <223> Xaa stands for 4-ImidazolylPpionyl-G
<400> 94
   Xaa Glu Gly Thr Phe Thr Ser Asp Leu Ser Lys Gln Leu Glu Glu Glu
   1 5 10 15
   Ala Val Arg Leu Phe Ile Glu Phe Leu Asn Xaa Gly Gly
   20 25
<210> 95
   <211> 28
   <212> PRT
   <213> Exendin Analog 93
<400> 95
   Ala Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 96
   <211> 28
   <212> PRT
   <213> Exendin Analog 94
<400> 96
   His Gly Ala Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 97
   <211> 28
   <212> PRT
   <213> Exendin Analog 95
<400> 97
   His Gly Glu Ala Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 98
   <211> 28
   <212> PRT
   <213> Exendin Analog 96
<400> 98
   His Gly Glu Gly Thr Phe Thr Ser Ala Leu Ser Lys Gln Leu Glu Glu
   1 5 10 15
   Glu Ala Val Arg Leu Phe Ile Glu Phe Leu Lys Asn
   20 25
<210> 99
   <211> 28
   <212> PRT
   <213> Exendin Analog 97
<400> 99
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 100
   <211> 28
   <212> PRT
   <213> Exendin Analog 98
<400> 100
   His Gly Ala Gly Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 101
   <211> 28
   <212> PRT
   <213> Exendin Analog 99
<400> 101
   His Gly Glu Ala Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 102
   <211> 28
   <212> PRT
   <213> Exendin Analog 100
<400> 102
   His Gly Glu Gly Thr Phe Thr Ser Ala Leu Ser Lys Gln Met Glu Glu
   1 5 10 15
   Glu Ala Val Arg Leu Phe Ile Glu Trp Leu Lys Asn
   20 25
<210> 103
   <211> 28
   <212> PRT
   <213> Exendin Analog 101
<400> 103
   His Gly Glu Gly Thr Phe Thr Ser Asp Ala Ser Lys Gln Met Glu Glu
   1 5 10 15
   Glu Ala Val Arg Leu Phe Ile Glu Trp Leu Lys Asn
   20 25
<210> 104
   <211> 28
   <212> PRT
   <213> Exendin Analog 102
<400> 104
   Ala Ala Glu Gly Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 105
   <211> 28
   <212> PRT
   <213> Exendin Analog 103
<400> 105
   Ala Ala Glu Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 106
   <211> 28
   <212> PRT
   <213> Exendin Analog 104
<400> 106
   Ala Gly Asp Gly Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 107
   <211> 28
   <212> PRT
   <213> Exendin Analog 105
<400> 107
   Ala Gly Asp Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 108
   <211> 28
   <212> PRT
   <213> Exendin Analog 106
<400> 108
   Ala Gly Asp Gly Ala Phe Thr Ser Asp Leu Ser Lys Gln Met Glu Glu
   1 5 10 15
   Glu Ala Val Arg Leu Phe Ile Glu Trp Leu Lys Asn
   20 25
<210> 109
   <211> 28
   <212> PRT
   <213> Exendin Analog 107
<400> 109
   Ala Gly Asp Gly Ala Phe Thr Ser Asp Leu Ser Lys Gln Leu Glu Glu
   1 5 10 15
   Glu Ala Val Arg Leu Phe Ile Glu Phe Leu Lys Asn
   20 25
<210> 110
   <211> 28
   <212> PRT
   <213> Exendin Analog 108
<220>
   <221> misc_feature
   <222> (6).. (6)
   <223> Xaa stands for Nala
<400> 110
   Ala Gly Asp Gly Thr Xaa Thr Ser Asp Leu Ser Lys Gln Met Glu Glu
   1 5 10 15
   Glu Ala Val Arg Leu Phe Ile Glu Trp Leu Lys Asn
   20 25
<210> 111
   <211> 28
   <212> PRT
   <213> Exendin Analog 109
<220>
   <221> misc_feature
   <222> (6)..(6)
   <223> Xaa stands for Nala
<400> 111
   Ala Gly Asp Gly Thr Xaa Thr Ser Asp Leu Ser Lys Gln Leu Glu Glu
   1 5 10 15
   Glu Ala Val Arg Leu Phe Ile Glu Phe Leu Lys Asn
   20 25
<210> 112
   <211> 28
   <212> PRT
   <213> Exendin Analog 110
<400> 112
   Ala Gly Asp Gly Thr Phe Ser Ser Asp Leu Ser Lys Gln Met Glu Glu
   1 5 10 15
   Glu Ala Val Arg Leu Phe Ile Glu Trp Leu Lys Asn
   20 25
<210> 113
   <211> 28
   <212> PRT
   <213> Exendin Analog 111
<400> 113
   Ala Gly Asp Gly Thr Phe Ser Ser Asp Leu Ser Lys Gln Leu Glu Glu
   1               5                   10                  15
   Glu Ala Val Arg Leu Phe Ile Glu Phe Leu Lys Asn
               20                  25
<210> 114
   <211> 28
   <212> PRT
   <213> Exendin Analog 112
<400> 114
   Ala Gly Asp Gly Thr Phe Thr Ala Asp Leu Ser Lys Gln Met Glu Glu
   1               5                   10                  15
   Glu Ala Val Arg Leu Phe Ile Glu Trp Leu Lys Asn
               20                  25
<210> 115
   <211> 28
   <212> PRT
   <213> Exendin Analog 113
<400> 115
   Ala Gly Asp Gly Thr Phe Thr Ala Asp Leu Ser Lys Gln Leu Glu Glu
   1               5                   10                  15
   Glu Ala Val Arg Leu Phe Ile Glu Phe Leu Lys Asn
               20                  25
<210> 116
   <211> 28
   <212> PRT
   <213> Exendin Analog 114
<400> 116
   Ala Gly Asp Gly Thr Phe Thr Ser Ala Leu Ser Lys Gln Met Glu Glu
   1               5                   10                  15
   Glu Ala Val Arg Leu Phe Ile Glu Trp Leu Lys Asn
               20                  25
<210> 117
   <211> 28
   <212> PRT
   <213> Exendin Analog 115
<400> 117
   Ala Gly Asp Gly Thr Phe Thr Ser Ala Leu Ser Lys Gln Leu Glu Glu
   1 5 10 15
   Glu Ala Val Arg Leu Phe Ile Glu Phe Leu Lys Asn
   20 25
<210> 118
   <211> 28
   <212> PRT
   <213> Exendin Analog 116
<400> 118
   Ala Gly Asp Gly Thr Phe Thr Ser Glu Leu Ser Lys Gln Met Glu Glu
   1 5 10 15
   Glu Ala Val Arg Leu Phe Ile Glu Trp Leu Lys Asn
   20 25
<210> 119
   <211> 28
   <212> PRT
   <213> Exendin Analog 117
<400> 119
   Ala Gly Asp Gly Thr Phe Thr Ser Glu Leu Ser Lys Gln Leu Glu Glu
   1 5 10 15
   Glu Ala Val Arg Leu Phe Ile Glu Phe Leu Lys Asn
   20 25
<210> 120
   <211> 28
   <212> PRT
   <213> Exendin Analog 118
<400> 120
   Ala Gly Asp Gly Thr Phe Thr Ser Asp Ala Ser Lys Gln Met Glu Glu
   1 5 10 15
   Glu Ala Val Arg Leu Phe Ile Glu Trp Leu Lys Asn
   20 25
<210> 121
   <211> 28
   <212> PRT
   <213> Exendin Analog 119
<400> 121
   Ala Gly Asp Gly Thr Phe Thr Ser Asp Ala Ser Lys Gln Leu Glu Glu
   1 5 10 15
   Glu Ala Val Arg Leu Phe Ile Glu Phe Leu Lys Asn
   20 25
<210> 122
   <211> 28
   <212> PRT
   <213> Exendin Analog 120
<220>
   <221> misc_feature
   <222> (10).. (10)
   <223> Xaa stands for Pgly
<400> 122
   Ala Gly Asp Gly Thr Phe Thr Ser Asp Xaa Ser Lys Gln Met Glu Glu
   1 5 10 15
   Glu Ala Val Arg Leu Phe Ile Glu Trp Leu Lys Asn
   20 25
<210> 123
   <211> 28
   <212> PRT
   <213> Exendin Analog 121
<220>
   <221> misc_feature
   <222> (10)..(10)
   <223> Xaa stands for Pgly
<400> 123
   Ala Gly Asp Gly Thr Phe Thr Ser Asp Xaa Ser Lys Gln Leu Glu Glu
   1 5 10 15
   Glu Ala Val Arg Leu Phe Ile Glu Phe Leu Lys Asn
   20 25
<210> 124
   <211> 28
   <212> PRT
   <213> Exendin Analog 122
<400> 124
   Ala Gly Asp Gly Thr Phe Thr Ser Asp Leu Ala Lys Gln Met Glu Glu
   1 5 10 15
   Glu Ala Val Arg Leu Phe Ile Glu Trp Leu Lys Asn
   20 25
<210> 125
   <211> 28
   <212> PRT
   <213> Exendin Analog 123
<400> 125
   Ala Gly Asp Gly Thr Phe Thr Ser Asp Leu Ala Lys Gln Leu Glu Glu
   1 5 10 15
   Glu Ala Val Arg Leu Phe Ile Glu Phe Leu Lys Asn
   20 25
<210> 126
   <211> 28
   <212> PRT
   <213> Exendin Analog 124
<400> 126
   Ala Gly Asp Gly Thr Phe Thr Ser Asp Leu Ser Ala Gln Met Glu Glu
   1 5 10 15
   Glu Ala Val Arg Leu Phe Ile Glu Trp Leu Lys Asn
   20 25
<210> 127
   <211> 28
   <212> PRT
   <213> Exendin Analog 125
<400> 127
   Ala Gly Asp Gly Thr Phe Thr Ser Asp Leu Ser Ala Gln Leu Glu Glu
   1 5 10 15
   Glu Ala Val Arg Leu Phe Ile Glu Phe Leu Lys Asn
   20 25
<210> 128
   <211> 28
   <212> PRT
   <213> Exendin Analog 126
<400> 128
   Ala Gly Asp Gly Thr Phe Thr Ser Asp Leu Ser Lys Ala Met Glu Glu
   1 5 10 15
   Glu Ala Val Arg Leu Phe Ile Glu Trp Leu Lys Asn
   20 25
<210> 129
   <211> 28
   <212> PRT
   <213> Exendin Analog 127
<400> 129
   Ala Gly Asp Gly Thr Phe Thr Ser Asp Leu Ser Lys Ala Leu Glu Glu
   1               5                   10                  15
   Glu Ala Val Arg Leu Phe Ile Glu Phe Leu Lys Asn
               20                  25
<210> 130
   <211> 28
   <212> PRT
   <213> Exendin Analog 128
<400> 130
   Ala Gly Asp Gly Thr Phe Thr Ser Asp Leu Ser Lys Gln Ala Glu Glu
   1               5                   10                  15
   Glu Ala Val Arg Leu Phe Ile Glu Trp Leu Lys Asn
               20                  25
<210> 131
   <211> 28
   <212> PRT
   <213> Exendin Analog 129
<400> 131
   Ala Gly Asp Gly Thr Phe Thr Ser Asp Leu Ser Lys Gln Ala Glu Glu
   1               5                   10                  15
   Glu Ala Val Arg Leu Phe Ile Glu Phe Leu Lys Asn
               20                  25
<210> 132
   <211> 28
   <212> PRT
   <213> Exendin Analog 130
<220>
   <221> misc_feature
   <222> (14)..(14)
   <223> Xaa stands for Pgly
<400> 132
   Ala Gly Asp Gly Thr Phe Thr Ser Asp Leu Ser Lys Gln Xaa Glu Glu
   1               5                   10                  15
   Glu Ala Val Arg Leu Phe Ile Glu Trp Leu Lys Asn
               20                  25
<210> 133
   <211> 28
   <212> PRT
   <213> Exendin Analog 131
<220>
   <221> misc_feature
   <222> (14)..(14)
   <223> Xaa stands for Pgly
<400> 133
   Ala Gly Asp Gly Thr Phe Thr Ser Asp Leu Ser Lys Gln Xaa Glu Glu
   1 5 10 15
   Glu Ala Val Arg Leu Phe Ile Glu Phe Leu Lys Asn
   20 25
<210> 134
   <211> 28
   <212> PRT
   <213> Exendin Analog 132
<400> 134
   Ala Gly Asp Gly Thr Phe Thr Ser Asp Leu Ser Lys Gln Met Ala Glu
   1 5 10 15
   Glu Ala Val Arg Leu Phe Ile Glu Trp Leu Lys Asn
   20 25
<210> 135
   <211> 28
   <212> PRT
   <213> Exendin Analog 133
<400> 135
   Ala Gly Asp Gly Thr Phe Thr Ser Asp Leu Ser Lys Gln Leu Ala Glu
   1 5 10 15
   Glu Ala Val Arg Leu Phe Ile Glu Phe Leu Lys Asn
   20 25
<210> 136
   <211> 28
   <212> PRT
   <213> Exendin Analog 134
<400> 136
   Ala Gly Asp Gly Thr Phe Thr Ser Asp Leu Ser Lys Gln Met Glu Ala
   1 5 10 15
   Glu Ala Val Arg Leu Phe Ile Glu Trp Leu Lys Asn
   20 25
<210> 137
   <211> 28
   <212> PRT
   <213> Exendin Analog 135
<400> 137
   Ala Gly Asp Gly Thr Phe Thr Ser Asp Leu Ser Lys Gln Leu Glu Ala
   1 5 10 15
   Glu Ala Val Arg Leu Phe Ile Glu Phe Leu Lys Asn
   20 25
<210> 138
   <211> 28
   <212> PRT
   <213> Exendin Analog 136
<400> 138
   Ala Gly Asp Gly Thr Phe Thr Ser Asp Leu Ser Lys Gln Met Glu Glu
   1 5 10 15
   Ala Ala Val Arg Leu Phe Ile Glu Trp Leu Lys Asn
   20 25
<210> 139
   <211> 28
   <212> PRT
   <213> Exendin Analog 137
<400> 139
   Ala Gly Asp Gly Thr Phe Thr Ser Asp Leu Ser Lys Gln Leu Glu Glu
   1 5 10 15
   Ala Ala Val Arg Leu Phe Ile Glu Phe Leu Lys Asn
   20 25
<210> 140
   <211> 28
   <212> PRT
   <213> Exendin Analog 138
<400> 140
   Ala Gly Asp Gly Thr Phe Thr Ser Asp Leu Ser Lys Gln Met Glu Glu
   1 5 10 15
   Glu Ala Ala Arg Leu Phe Ile Glu Trp Leu Lys Asn
   20 25
<210> 141
   <211> 28
   <212> PRT
   <213> Exendin Analog 139
<400> 141
   Ala Gly Asp Gly Thr Phe Thr Ser Asp Leu Ser Lys Gln Leu Glu Glu
   1 5 10 15
   Glu Ala Ala Arg Leu Phe Ile Glu Phe Leu Lys Asn
   20 25
<210> 142
   <211> 28
   <212> PRT
   <213> Exendin Analog 140
<400> 142
   Ala Gly Asp Gly Thr Phe Thr Ser Asp Leu Ser Lys Gln Met Glu Glu
   1               5                   10                  15
   Glu Ala Val Ala Leu Phe Ile Glu Trp Leu Lys Asn
               20                  25
<210> 143
   <211> 28
   <212> PRT
   <213> Exendin Analog 141
<400> 143
   Ala Gly Asp Gly Thr Phe Thr Ser Asp Leu Ser Lys Gln Leu Glu Glu
   1               5                   10                  15
   Glu Ala Val Ala Leu Phe Ile Glu Phe Leu Lys Asn
               20                  25
<210> 144
   <211> 28
   <212> PRT
   <213> Exendin Analog 142
<400> 144
   Ala Gly Asp Gly Thr Phe Thr Ser Asp Leu Ser Lys Gln Met Glu Glu
   1               5                   10                  15
   Glu Ala Val Arg Ala Phe Ile Glu Trp Leu Lys Asn
               20                  25
<210> 145
   <211> 28
   <212> PRT
   <213> Exendin Analog 143
<400> 145
   Ala Gly Asp Gly Thr Phe Thr Ser Asp Leu Ser Lys Gln Leu Glu Glu
   1               5                   10                  15
   Glu Ala Val Arg Ala Phe Ile Glu Phe Leu Lys Asn
               20                  25
<210> 146
   <211> 28
   <212> PRT
   <213> Exendin Analog 144
<220>
   <221> misc_feature
   <222> (22)..(22)
   <223> Xaa stands for Nala
<400> 146
   Ala Gly Asp Gly Thr Phe Thr Ser Asp Leu Ser Lys Gln Met Glu Glu
   1 5 10 15
   Glu Ala Val Arg Leu Xaa Ile Glu Trp Leu Lys Asn
   20 25
<210> 147
   <211> 28
   <212> PRT
   <213> Exendin Analog 145
<220>
   <221> misc_feature
   <222> (22)..(22)
   <223> Xaa stands for Nala
<400> 147
   Ala Gly Asp Gly Thr Phe Thr Ser Asp Leu Ser Lys Gln Leu Glu Glu
   1 5 10 15
   Glu Ala Val Arg Leu Xaa Ile Glu Phe Leu Lys Asn
   20 25
<210> 148
   <211> 28
   <212> PRT
   <213> Exendin Analog 146
<400> 148
   Ala Gly Asp Gly Thr Phe Thr Ser Asp Leu Ser Lys Gln Met Glu Glu
   1 5 10 15
   Glu Ala Val Arg Leu Phe Val Glu Trp Leu Lys Asn
   20 25
<210> 149
   <211> 28
   <212> PRT
   <213> Exendin Analog 147
<400> 149
   Ala Gly Asp Gly Thr Phe Thr Ser Asp Leu Ser Lys Gln Leu Glu Glu
   1 5 10 15
   Glu Ala Val Arg Leu Phe Val Glu Phe Leu Lys Asn
   20 25
<210> 150
   <211> 28
   <212> PRT
   <213> Exendin Analog 148
<220>
   <221> misc_feature
   <222> (23)..(23)
   <223> Xaa stands for Tgly
<400> 150
   Ala Gly Asp Gly Thr Phe Thr Ser Asp Leu Ser Lys Gln Met Glu Glu
   1               5                   10                  15
   Glu Ala Val Arg Leu Phe Xaa Glu Trp Leu Lys Asn
               20                  25
<210> 151
   <211> 28
   <212> PRT
   <213> Exendin Analog 149
<220>
   <221> misc_feature
   <222> (23)..(23)
   <223> Xaa stands for Tgly
<400> 151
   Ala Gly Asp Gly Thr Phe Thr Ser Asp Leu Ser Lys Gln Leu Glu Glu
   1               5                   10                  15
   Glu Ala Val Arg Leu Phe Xaa Glu Phe Leu Lys Asn
               20                  25
<210> 152
   <211> 28
   <212> PRT
   <213> Exendin Analog 150
<400> 152
   Ala Gly Asp Gly Thr Phe Thr Ser Asp Leu Ser Lys Gln Met Glu Glu
   1               5                   10                  15
   Glu Ala Val Arg Leu Phe Ile Asp Trp Leu Lys Asn
               20                  25
<210> 153
   <211> 28
   <212> PRT
   <213> Exendin Analog 151
<400> 153
   Ala Gly Asp Gly Thr Phe Thr Ser Asp Leu Ser Lys Gln Leu Glu Glu
   1 5 10 15
   Glu Ala Val Arg Leu Phe Ile Asp Phe Leu Lys Asn
   20 25
<210> 154
   <211> 28
   <212> PRT
   <213> Exendin Analog 152
<400> 154
   Ala Gly Asp Gly Thr Phe Thr Ser Asp Leu Ser Lys Gln Met Glu Glu
   1 5 10 15
   Glu Ala Val Arg Leu Phe Ile Glu Ala Leu Lys Asn
   20 25
<210> 155
   <211> 28
   <212> PRT
   <213> Exendin Analog 153
<400> 155
   Ala Gly Asp Gly Thr Phe Thr Ser Asp Leu Ser Lys Gln Leu Glu Glu
   1 5 10 15
   Glu Ala Val Arg Leu Phe Ile Glu Ala Leu Lys Asn
   20 25
<210> 156
   <211> 28
   <212> PRT
   <213> Exendin Analog 154
<400> 156
   Ala Gly Asp Gly Thr Phe Thr Ser Asp Leu Ser Lys Gln Met Glu Glu
   1 5 10 15
   Glu Ala Val Arg Leu Phe Ile Glu Trp Ala Lys Asn
   20 25
<210> 157
   <211> 28
   <212> PRT
   <213> Exendin Analog 155
<400> 157
   Ala Gly Asp Gly Thr Phe Thr Ser Asp Leu Ser Lys Gln Leu Glu Glu
   1 5 10 15
   Glu Ala Val Arg Leu Phe Ile Glu Phe Ala Lys Asn
   20 25
<210> 158
   <211> 28
   <212> PRT
   <213> Exendin Analog 156
<400> 158
   Ala Gly Asp Gly Thr Phe Thr Ser Asp Leu Ser Lys Gln Met Glu Glu
   1 5 10 15
   Glu Ala Val Arg Leu Phe Ile Glu Trp Leu Ala Asn
   20 25
<210> 159
   <211> 28
   <212> PRT
   <213> Exendin Analog 157
<400> 159
   Ala Gly Asp Gly Thr Phe Thr Ser Asp Leu Ser Lys Gln Leu Glu Glu
   1 5 10 15
   Glu Ala Val Arg Leu Phe Ile Glu Phe Leu Ala Asn
   20 25
<210> 160
   <211> 28
   <212> PRT
   <213> Exendin Analog 158
<400> 160
   Ala Gly Asp Gly Thr Phe Thr Ser Asp Leu Ser Lys Gln Met Glu Glu
   1 5 10 15
   Glu Ala Val Arg Leu Phe Ile Glu Trp Leu Lys Ala
   20 25
<210> 161
   <211> 28
   <212> PRT
   <213> Exendin Analog 159
<400> 161
   Ala Gly Asp Gly Thr Phe Thr Ser Asp Leu Ser Lys Gln Leu Glu Glu
   1 5 10 15
   Glu Ala Val Arg Leu Phe Ile Glu Phe Leu Lys Ala
   20 25
<210> 162
   <211> 38
   <212> PRT
   <213> Exendin Analog 160
<400> 162
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro
   35
<210> 163
   <211> 38
   <212> PRT
   <213> Exendin Analog 161
<400> 163
   His Gly Ala Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro
   35
<210> 164
   <211> 37
   <212> PRT
   <213> Exendin Analog 162
<400> 164
   His Gly Glu Ala Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro
   35
<210> 165
   <211> 36
   <212> PRT
   <213> Exendin Analog 163
<400> 165
   His Gly Glu Gly Thr Phe Thr Ser Ala Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro
   35
<210> 166
   <211> 36
   <212> PRT
   <213> Exendin Analog 164
<400> 166
   Ala Gly Glu Gly Thr Phe Thr Ser Asp Ala Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro
   35
<210> 167
   <211> 35
   <212> PRT
   <213> Exendin Analog 165
<400> 167
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala
   35
<210> 168
   <211> 35
   <212> PRT
   <213> Exendin Analog 166
<400> 168
   His Gly Ala Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala
   35
<210> 169
   <211> 34
   <212> PRT
   <213> Exendin Analog 167
<400> 169
   His Gly Glu Ala Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly
<210> 170
   <211> 32
   <212> PRT
   <213> Exendin Analog 168
<400> 170
   His Gly Glu Gly Thr Phe Thr Ser Ala Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
<210> 171
   <211> 32
   <212> PRT
   <213> Exendin Analog 169
<400> 171
   Ala Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
<210> 172
   <211> 32
   <212> PRT
   <213> Exendin Analog 170
<400> 172
   His Gly Ala Gly Thr Phe Thr Ser Asp Leu Ser Lys Gln Leu Glu Glu
   1               5                   10                  15
   Glu Ala Val Arg Leu Phe Ile Glu Phe Leu Lys Asn Gly Gly Pro Ser
               20                  25                  30
<210> 173
   <211> 31
   <212> PRT
   <213> Exendin Analog 171
<400> 173
   His Gly Glu Ala Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro
   20 25 30
<210> 174
   <211> 30
   <212> PRT
   <213> Exendin Analog 172
<400> 174
   His Gly Glu Gly Thr Phe Thr Ser Ala Leu Ser Lys Gln Leu Glu Glu
   1 5 10 15
   Glu Ala Val Arg Leu Phe Ile Glu Phe Leu Lys Asn Gly Gly
   20 25 30
<210> 175
   <211> 29
   <212> PRT
   <213> Exendin Analog 173
<400> 175
   Ala Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly
   20 25
<210> 176
   <211> 38
   <212> PRT
   <213> Exendin Analog 174
<220>
   <221> misc-feature
   <222> (31) .. (31)
   <223> Xaa stands for tPro
<220>
   <221> misc_feature
   <222> (36) .. (38)
   <223> Xaa stands for tPro
<400> 176
   His Gly Ala Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Xaa Ser
   20 25 30
   Ser Gly Ala Xaa Xaa Xaa
   35
<210> 177
   <211> 38
   <212> PRT
   <213> Exendin Analog 175
<220>
   <221> misc_feature
   <222> (36) .. (38)
   <223> Xaa stands for tPro
<400> 177
   His Gly Glu Ala Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Xaa Xaa Xaa
   35
<210> 178
   <211> 37
   <212> PRT
   <213> Exendin Analog 176
<220>
   <221> misc_feature
   <222> (31) .. (31)
   <223> Xaa stands for Nme
<220>
   <221> misc _feature
   <222> (36) .. (37)
   <223> Xaa stands for Nme
<400> 178
   His Gly Glu Gly Thr Phe Thr Ser Ala Leu Ser Lys Gln Met Glu Glu
   1 5 10 15
   Glu Ala Val Arg Leu Phe Ile Glu Trp Leu Lys Asn Gly Gly Xaa Ser
   20 25 30
   Ser Gly Ala Xaa Xaa
   35
<210> 179
   <211> 36
   <212> PRT
   <213> Exendin Analog 177
<220>
   <221> misc_feature
   <222> (31) .. (31)
   <223> Xaa stands for tPro
<220>
   <221> misc_feature
   <222> (36) .. (36)
   <223> Xaa stands for tPro
<400> 179
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Xaa Ser
   20 25 30
   Ser Gly Ala Xaa
   35
<210> 180
   <211> 35
   <212> PRT
   <213> Exendin Analog 178
<400> 180
   His Gly Ala Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala
   35
<210> 181
   <211> 30
   <212> PRT
   <213> Exendin Analog 179
<400> 181
   His Gly Asp Ala Thr Phe Thr Ser Asp Leu Ser Lys Gln Met Glu Glu
   1 5 10 15
   Glu Ala Val Arg Leu Phe Ile Glu Trp Leu Lys Asn Gly Gly
   20 25 30
<210> 182
   <211> 39
   <212> PRT
   <213> Exendin Analog 180
<400> 182
   Ala Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 183
   <211> 39
   <212> PRT
   <213> Exendin Analog 181
<400> 183
   Ala Gly Ala Gly Thr Phe Thr Ser Asp Leu Ser Lys Gln Leu Glu Glu
   1               5                   10                  15
   Glu Ala Val Arg Leu Phe Ile Glu Phe Leu Lys Asn Gly Gly Pro Ser
               20                  25                  30
   Ser Gly Ala Pro Pro Pro Ser
           35
<210> 184
   <211> 27
   <212> PRT
   <213> Exendin Analog 182
<220>
   <221> misc_feature
   <222> (1) .. (1)
   <223> Xaa stands for 4-ImidazolylPpionyl-G
<220>
   <221> misc_feature
   <222> (26) .. (26)
   <223> Xaa stands for Lys-NH(epsilon)octanoyl
<400> 184
   Xaa Glu Gly Thr Phe Thr Ser Ala Leu Ser Lys Gln Met Glu Glu Glu
   1               5                   10                  15
   Ala Val Arg Leu Phe Ile Glu Trp Leu Xaa Asn
               20                  25
<210> 185
   <211> 27
   <212> PRT
   <213> Exendin Analog 183
<220>
   <221> misc_feature
   <222> (1) .. (1)
   <223> Xaa stands for 4-ImidazolylPpionyl-G
<220>
   <221> misc_feature
   <222> (26) .. (26)
   <223> Xaa stands for Lys-NH(epsilon)octanoyl
<400> 185
   Xaa Glu Gly Thr Phe Thr Ser Ala Leu Ser Lys Gln Leu Glu Glu Glu
   1 5 10 15
   Ala Val Arg Leu Phe Ile Glu Phe Leu Xaa Asn
   20 25
<210> 186
   <211> 29
   <212> PRT
   <213> Exendin Analog 184
<220>
   <221> misc_feature
   <222> (1) .. (1)
   <223> Xaa stands for 4-ImidazolylPpionyl-G
<220>
   <221> misc_feature
   <222> (26) .. (26)
   <223> Xaa stands for Lys-NH(epsilon)octanoyl
<400> 186
   Xaa Glu Gly Thr Phe Thr Ser Ala Leu Ser Lys Gln Met Glu Glu Glu
   1 5 10 15
   Ala Val Arg Leu Phe Ile Glu Trp Leu Xaa Asn Gly Gly
   20 25
<210> 187
   <211> 29
   <212> PRT
   <213> Exendin Analog 185
<220>
   <221> misc_feature
   <222> (1) .. (1)
   <223> Xaa stands for 4-ImidazolylPpionyl-G
<220>
   <221> misc_feature
   <222> (26) .. (26)
   <223> Xaa stands for Lys-NH(epsilon)octanoyl
<400> 187
   Xaa Glu Gly Thr Phe Thr Ser Ala Leu Ser Lys Gln Leu Glu Glu Glu
   1               5                   10                  15
   Ala Val Arg Leu Phe Ile Glu Phe Leu Xaa Asn Gly Gly
               20                  25
<210> 188
   <211> 27
   <212> PRT
   <213> Exendin Analog 186
<220>
   <221> misc_feature
   <222> (1) .. (1)
   <223> Xaa stands for 4-ImidazolylPpionyl-G
<220>
   <221> misc_feature
   <222> (27) .. (27)
   <223> Xaa stands for Lys-NH(epsilon)octanoyl
<400> 188
   Xaa Glu Gly Thr Phe Thr Ser Ala Leu Ser Lys Gln Met Glu Glu Glu
   1               5                   10                  15
   Ala Val Arg Leu Phe Ile Glu Trp Leu Asn Xaa
               20                  25
<210> 189
   <211> 27
   <212> PRT
   <213> Exendin Analog 187
<220>
   <221> misc_feature
   <222> (1) .. (1)
   <223> Xaa stands for 4-ImidazolylPpionyl-G
<220>
   <221> misc_feature
   <222> (27) .. (27)
   <223> Xaa stands for Lys-NH(epsilon)octanoyl
<400> 189
   Xaa Glu Gly Thr Phe Thr Ser Ala Leu Ser Lys Gln Leu Glu Glu Glu
   1               5                   10                  15
   Ala Val Arg Leu Phe Ile Glu Phe Leu Asn Xaa
               20                  25
<210> 190
   <211> 29
   <212> PRT
   <213> Exendin Analog 188
<220>
   <221> misc_feature
   <222> (1) .. (1)
   <223> Xaa stands for 4-ImidazolylPpionyl-G
<220>
   <221> misc_feature
   <222> (27) .. (27)
   <223> Xaa stands for Lys-NH(epsilon)octanoyl
<400> 190
   Xaa Glu Gly Thr Phe Thr Ser Ala Leu Ser Lys Gln Met Glu Glu Glu
   1               5                   10                  15
   Ala Val Arg Leu Phe Ile Glu Trp Leu Asn Xaa Gly Gly
               20                  25
<210> 191
   <211> 29
   <212> PRT
   <213> Exendin Analog 189
<220>
   <221> misc_feature
   <222> (1) .. (1)
   <223> Xaa stands for 4-ImidazolylPpionyl-G
<220>
   <221> misc_feature
   <222> (27) .. (27)
   <223> Xaa stands for Lys-NH(epsilon)octanoyl
<400> 191
   Xaa Glu Gly Thr Phe Thr Ser Ala Leu Ser Lys Gln Leu Glu Glu Glu
   1               5                   10                  15
   Ala Val Arg Leu Phe Ile Glu Phe Leu Asn Xaa Gly Gly
               20                  25
<210> 192
   <211> 28
   <212> PRT
   <213> Exendin Analog 190
<220>
   <221> misc_feature
   <222> (27) .. (27)
   <223> Xaa stands for Lys-NH(epsilon)octanoyl
<400> 192
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Xaa Asn
   20 25
<210> 193
   <211> 28
   <212> PRT
   <213> Exendin Analog 191
<220>
   <221> misc_feature
   <222> (27) .. (27)
   <223> Xaa stands for Lys-NH(epsilon)octanoyl
<400> 193
   Ala Gly Glu Gly Thr Phe Thr Ser Asp Leu Ser Lys Gln Leu Glu Glu
   1 5 10 15
   Glu Ala Val Arg Leu Phe Ile Glu Phe Leu Xaa Asn
   20 25
<210> 194
   <211> 30
   <212> PRT
   <213> Exendin Analog 192
<220>
   <221> misc_feature
   <222> (27) .. (27)
   <223> Xaa stands for Lys-NH(epsilon)octanoyl
<400> 194
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Xaa Asn Gly Gly
   20 25 30
<210> 195
   <211> 30
   <212> PRT
   <213> Exendin Analog 193
<220>
   <221> misc_feature
   <222> (27) .. (27)
   <223> Xaa stands for Lys-NH(epsilon)octanoyl
<400> 195
   Ala Gly Glu Gly Thr Phe Thr Ser Asp Leu Ser Lys Gln Leu Glu Glu
   1 5 10 15
   Glu Ala Val Arg Leu Phe Ile Glu Phe Leu Xaa Asn Gly Gly
   20 25 30
<210> 196
   <211> 28
   <212> PRT
   <213> Exendin Analog 194
<220>
   <221> misc_feature
   <222> (28) .. (28)
   <223> Xaa stands for Lys-NH(epsilon)octanoyl
<400> 196
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Asn Xaa
   20 25
<210> 197
   <211> 28
   <212> PRT
   <213> Exendin Analog 195
<220>
   <221> misc_feature
   <222> (28) .. (28)
   <223> Xaa stands for Lys-NH(epsilon)octanoyl
<400> 197
   Ala Gly Glu Gly Thr Phe Thr Ser Asp Leu Ser Lys Gln Leu Glu Glu
   1 5 10 15
   Glu Ala Val Arg Leu Phe Ile Glu Phe Leu Asn Xaa
   20 25
<210> 198
   <211> 30
   <212> PRT
   <213> Exendin Analog 196
<220>
   <221> misc_feature
   <222> (28) .. (28)
   <223> Xaa stands for Lys-NH(epsilon)octanoyl
<400> 198
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Asn Xaa Gly Gly
   20 25 30
<210> 199
   <211> 30
   <212> PRT
   <213> Exendin Analog 197
<220>
   <221> misc_feature
   <222> (28) .. (28)
   <223> Xaa stands for Lys-NH(epsilon)octanoyl
<400> 199
   Ala Gly Glu Gly Thr Phe Thr Ser Asp Leu Ser Lys Gln Leu Glu Glu
   1 5 10 15
   Glu Ala Val Arg Leu Phe Ile Glu Phe Leu Asn Xaa Gly Gly
   20 25 30
<210> 200
   <211> 39
   <212> PRT
   <213> Exendin Analog 198
<220>
   <221> misc_feature
   <222> (12) .. (12)
   <223> Lys is Pegylated
<400> 200
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 201
   <211> 39
   <212> PRT
   <213> Exendin Analog 199
<220>
   <221> misc_feature
   <222> (27) .. (27)
   <223> Lys is Pegylated
<400> 201
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 202
   <211> 39
   <212> PRT
   <213> Exendin Analog 200
<220>
   <221> misc_feature
   <222> (2) .. (2)
   <223> Lys is Pegylated
<400> 202
   His Lys Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 203
   <211> 39
   <212> PRT
   <213> Exendin Analog 201
<220>
   <221> misc_feature
   <222> (5) .. (5)
   <223> Lys is Pegylated
<400> 203
   His Gly Glu Gly Lys Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 204
   <211> 39
   <212> PRT
   <213> Exendin Analog 202
<220>
   <221> misc_feature
   <222> (8) .. (8)
   <223> Lys is Pegylated
<400> 204
   His Gly Glu Gly Thr Phe Thr Lys Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 205
   <211> 39
   <212> PRT
   <213> Exendin Analog 203
<220>
   <221> misc_feature
   <222> (10) .. (10)
   <223> Lys is Pegylated
<400> 205
   His Gly Glu Gly Thr Phe Thr Ser Asp Lys Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 206
   <211> 38
   <212> PRT
   <213> Exendin Analog 204
<220>
   <221> misc_feature
   <222> (11) .. (11)
   <223> Lys is Pegylated
<400> 206
   His Gly Glu Gly Thr Phe Thr Ser Asp Leu Lys Lys Gln Met Glu Glu
   1 5 10 15
   Glu Ala Val Arg Leu Phe Ile Glu Trp Leu Lys Gly Gly Pro Ser Ser
   20 25 30
   Gly Ala Pro Pro Pro Ser
   35
<210> 207
   <211> 39
   <212> PRT
   <213> Exendin Analog 205
<220>
   <221> misc_feature
   <222> (13) .. (13)
   <223> Lys is Pegylated
<400> 207
   His Gly Glu Gly Thr Phe Thr Ser Asp Leu Ser Lys Lys Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 208
   <211> 39
   <212> PRT
   <213> Exendin Analog 206
<220>
   <221> misc_feature
   <222> (16) .. (16)
   <223> Lys is Pegylated
<400> 208
   His Gly Glu Gly Thr Phe Thr Ser Asp Leu Ser Lys Gln Met Glu Lys
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 209
   <211> 39
   <212> PRT
   <213> Exendin Analog 207
<220>
   <221> misc_feature
   <222> (17) .. (17)
   <223> Lys is Pegylated
<400> 209
   His Gly Glu Gly Thr Phe Thr Ser Asp Leu Ser Lys Gln Met Glu Glu
   1 5 10 15
   Lys Ala Val Arg Leu Phe Ile Glu Trp Leu Lys Asn Gly Gly Pro Ser
   20 25 30
   Ser Gly Ala Pro Pro Pro Ser
   35
<210> 210
   <211> 39
   <212> PRT
   <213> Exendin Analog 208
<220>
   <221> misc-feature
   <222> (19) .. (19)
   <223> Lys is Pegylated
<400> 210
   His Gly Glu Gly Thr Phe Thr Ser Asp Leu Ser Lys Gln Met Glu Glu
   1 5 10 15
   Glu Ala Lys Arg Leu Phe Ile Glu Trp Leu Lys Asn Gly Gly Pro Ser
   20 25 30
   Ser Gly Ala Pro Pro Pro Ser
   35
<210> 211
   <211> 39
   <212> PRT
   <213> Exendin Analog 209
<220>
   <221> misc_feature
   <222> (21) .. (21)
   <223> Lys is Pegylated
<400> 211
   His Gly Glu Gly Thr Phe Thr Ser Asp Leu Ser Lys Gln Met Glu Glu
   1 5 10 15
   Glu Ala Val Arg Ser Phe Ile Glu Trp Leu Lys Asn Gly Gly Pro Ser
   20 25 30
   Ser Gly Ala Pro Pro Pro Ser
   35
<210> 212
   <211> 39
   <212> PRT
   <213> Exendin Analog 210
<220>
   <221> misc_feature
   <222> (24) .. (24)
   <223> Lys is Pegylated
<400> 212
   His Gly Glu Gly Thr Phe Thr Ser Asp Leu Ser Lys Gln Met Glu Glu
   1 5 10 15
   Glu Ala Val Arg Leu Phe Ile Lys Trp Leu Lys Asn Gly Gly Pro Ser
   20 25 30
   Ser Gly Ala Pro Pro Pro Ser
   35
<210> 213
   <211> 39
   <212> PRT
   <213> Exendin Analog 211
<220>
   <221> misc_feature
   <222> (25) .. (25)
   <223> Lys is Pegylated
<400> 213
   His Gly Glu Gly Thr Phe Thr Ser Asp Leu Ser Lys Gln Met Glu Glu
   1 5 10 15
   Glu Ala Val Arg Leu Phe Ile Glu Lys Leu Lys Asn Gly Gly Pro Ser
   20 25 30
   Ser Gly Ala Pro Pro Pro Ser
   35
<210> 214
   <211> 39
   <212> PRT
   <213> Exendin Analog 212
<220>
   <221> misc_feature
   <222> (28) .. (28)
   <223> Lys is Pegylated
<400> 214
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Lys Gly Gly Pro Ser
   20 25 30
   Ser Gly Ala Pro Pro Pro Ser
   35
<210> 215
   <211> 39
   <212> PRT
   <213> Exendin Analog 213
<220>
   <221> misc_feature
   <222> (29) .. (29)
   <223> Lys is Pegylated
<400> 215
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Lys Gly Pro Ser
   20 25 30
   Ser Gly Ala Pro Pro Pro Ser
   35
<210> 216
   <211> 39
   <212> PRT
   <213> Exendin Analog 214
<220>
   <221> misc_feature
   <222> (30) .. (30)
   <223> Lys is Pegylated
<400> 216
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Lys Pro Ser
   20 25 30
   Ser Gly Ala Pro Pro Pro Ser
   35
<210> 217
   <211> 39
   <212> PRT
   <213> Exendin Analog 215
<220>
   <221> misc_feature
   <222> (1) .. (1)
   <223> His is Acylated
<400> 217
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 218
   <211> 39
   <212> PRT
   <213> Exendin Analog 216
<220>
   <221> misc_feature
   <222> (1) .. (1)
   <223> His is Alkylated
<400> 218
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 219
   <211> 39
   <212> PRT
   <213> Exendin Analog 217
<220>
   <221> misc_feature
   <222> (27) .. (27)
   <223> Lys is Pegylated
<400> 219
   His Gly Glu Gly Thr Phe Thr Ser Asp Leu Ser Arg Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 220
   <211> 39
   <212> PRT
   <213> Exendin Analog 218
<220>
   <221> misc_feature
   <222> (12) .. (12)
   <223> Lys is Pegylated
<400> 220
   His Gly Glu Gly Thr Phe Thr Ser Asp Leu Ser Lys Gln Met Glu Glu
   1 5 10 15
   Glu Ala Val Arg Leu Phe Ile Glu Trp Leu Arg Asn Gly Gly Pro Ser
   20 25 30
   Ser Gly Ala Pro Pro Pro Ser
   35
<210> 221
   <211> 39
   <212> PRT
   <213> Exendin Analog 219
<400> 221
   His Gly Glu Gly Thr Phe Thr Ser Asp Leu Ser Arg Gln Met Glu Glu
   1 5 10 15
   Glu Ala Val Arg Leu Phe Ile Glu Trp Leu Arg Asn Gly Gly Pro Ser
   20 25 30
   Ser Gly Ala Pro Pro Pro Ser
   35
<210> 222
   <211> 39
   <212> PRT
   <213> Exendin Analog 220
<220>
   <221> misc_feature
   <222> (29) .. (29)
   <223> Lys is Pegylated
<400> 222
   His Gly Glu Gly Thr Phe Thr Ser Asp Leu Ser Arg Gln Met Glu Glu
   1 5 10 15
   Glu Ala Val Arg Leu Phe Ile Glu Trp Leu Arg Asn Gly Gly Pro Ser
   20 25 30
   Ser Gly Ala Pro Pro Pro Lys
   35
<210> 223
   <211> 39
   <212> PRT
   <213> Exendin Analog 221
<220>
   <221> misc_feature
   <222> (30) .. (30)
   <223> Lys is Pegylated
<400> 223
   His Gly Glu Gly Thr Phe Thr Ser Asp Leu Ser Arg Gln Met Glu Glu
   1 5 10 15
   Glu Ala Val Arg Leu Phe Ile Glu Trp Leu Arg Asn Gly Lys Pro Ser
   20 25 30
   Ser Gly Ala Pro Pro Pro Ser
   35
<210> 224
   <211> 39
   <212> PRT
   <213> Exendin Analog 222
<220>
   <221> misc_feature
   <222> (27) .. (27)
   <223> Lys is Pegylated
<400> 224
   His Gly Glu Gly Thr Phe Thr Ser Asp Leu Ser Arg Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 225
   <211> 39
   <212> PRT
   <213> Exendin Analog 223
<220>
   <221> misc_feature
   <222> (12) .. (12)
   <223> Lys is Pegylated
<400> 225
   His Gly Glu Gly Thr Phe Thr Ser Asp Leu Ser Lys Gln Met Glu Glu
   1 5 10 15
   Glu Ala Val Arg Leu Phe Ile Glu Trp Leu Arg Asn Gly Gly Pro Ser
   20 25 30
   Ser Gly Ala Pro Pro Pro Ser
   35
<210> 226
   <211> 39
   <212> PRT
   <213> Exendin Analog 224
<220>
   <221> misc_feature
   <222> (1) .. (1)
   <223> His is Pegylated
<400> 226
   His Gly Glu Gly Thr Phe Thr Ser Asp Leu Ser Arg Gln Met Glu Glu
   1               5                   10                  15
   Glu Ala Val Arg Leu Phe Ile Glu Trp Leu Arg Asn Gly Gly Pro Ser
               20                  25                  30
   Ser Gly Ala Pro Pro Pro Ser
           35
<210> 227
   <211> 39
   <212> PRT
   <213> Exendin Analog 225
<220>
   <221> misc_feature
   <222> (1) .. (1)
   <223> His is Pegylated
<400> 227
   His Gly Glu Gly Thr Phe Thr Ser Asp Leu Ser Arg Gln Met Glu Glu
   1               5                   10                  15
   Glu Ala Val Arg Leu Phe Ile Glu Trp Leu Arg Asn Gly Gly Pro Ser
               20                  25                  30
   Ser Gly Ala Pro Pro Pro Ser
           35
<210> 228
   <211> 39
   <212> PRT
   <213> Exendin Analog 226
<220>
   <221> misc_feature
   <222> (39) .. (39)
   <223> Lys is Pegylated
<400> 228
   His Gly Glu Gly Thr Phe Thr Ser Asp Leu Ser Arg Gln Met Glu Glu
   1 5 10 15
   Glu Ala Val Arg Leu Phe Ile Glu Trp Leu Arg Asn Gly Gly Pro Ser
   20 25 30
   Ser Gly Ala Pro Pro Pro Lys
   35
<210> 229
   <211> 39
   <212> PRT
   <213> Exendin Analog 227
<220>
   <221> misc_feature
   <222> (30) .. (30)
   <223> Lys is Pegylated
<400> 229
   His Gly Glu Gly Thr Phe Thr Ser Asp Leu Ser Arg Gln Met Glu Glu
   1 5 10 15
   Glu Ala Val Arg Leu Phe Ile Glu Trp Leu Arg Asn Gly Lys Pro Ser
   20 25 30
   Ser Gly Ala Pro Pro Pro Ser
   35
<210> 230
   <211> 39
   <212> PRT
   <213> Exendin Analog 228
<400> 230
   His Gly Glu Gly Thr Phe Thr Ser Asp Leu Ser Lys Gln Arg Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 231
   <211> 39
   <212> PRT
   <213> Exendin Analog 229
<400> 231
   His Gly Glu Gly Thr Phe Thr Ser Asp Leu Ser Lys Gln Arg Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser
   35
<210> 232
   <211> 39
   <212> PRT
   <213> Exendin Analog 230
<400> 232
   His Gly Glu Gly Thr Phe Thr Ser Asp Leu Ser Lys Gln Arg Glu Glu
   1 5 10 15
   Glu Ala Val Lys Leu Phe Ile Glu Trp Leu Lys Asn Gly Gly Pro Ser
   20 25 30
   Ser Gly Ala Pro Pro Pro Ser
   35
<210> 233
   <211> 40
   <212> PRT
   <213> Exendin Analog 231
<400> 233
   His Gly Glu Gly Thr Phe Thr Ser Asp Leu Ser Lys Gln Arg Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Arg
   35 40
<210> 234
   <211> 39
   <212> PRT
   <213> Exendin Analog 232
<400> 234
   His Gly Glu Gly Thr Phe Thr Ser Asp Leu Ser Lys Gln Arg Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 235
   <211> 40
   <212> PRT
   <213> Exendin Analog 233
<400> 235
   His Gly Glu Gly Thr Phe Thr Ser Asp Leu Ser Lys Gln Arg Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Lys
   35 40
<210> 236
   <211> 40
   <212> PRT
   <213> Exendin Analog 234
<400> 236
   His Gly Glu Gly Thr Phe Thr Ser Asp Leu Ser Lys Gln Arg Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser Arg
   35 40
<210> 237
   <211> 39
   <212> PRT
   <213> Exendin Analog 235
<400> 237
   His Gly Glu Gly Thr Phe Thr Ser Asp Leu Ser Lys Gln Arg Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser
   35
<210> 238
   <211> 40
   <212> PRT
   <213> Exendin Analog 236
<400> 238
   His Gly Glu Gly Thr Phe Thr Ser Asp Leu Ser Lys Gln Arg Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser Lys
   35 40
<210> 239
   <211> 40
   <212> PRT
   <213> Exendin Analog 237
<400> 239
   His Gly Glu Gly Thr Phe Thr Ser Asp Leu Ser Lys Gln Arg Glu Glu
   1 5 10 15
   Glu Ala Val Lys Leu Phe Ile Glu Trp Leu Lys Asn Gly Gly Pro Ser
   20 25 30
   Ser Gly Ala Pro Pro Pro Ser Arg
   35 40
<210> 240
   <211> 39
   <212> PRT
   <213> Exendin Analog 238
<400> 240
   His Gly Glu Gly Thr Phe Thr Ser Asp Leu Ser Lys Gln Arg Glu Glu
   1 5 10 15
   Glu Ala Val Lys Leu Phe Ile Glu Trp Leu Lys Asn Gly Gly Pro Ser
   20 25 30
   Ser Gly Ala Pro Pro Pro Ser
   35
<210> 241
   <211> 40
   <212> PRT
   <213> Exendin Analog 239
<400> 241
   His Gly Glu Gly Thr Phe Thr Ser Asp Leu Ser Lys Gln Arg Glu Glu
   1 5 10 15
   Glu Ala Val Lys Leu Phe Ile Glu Trp Leu Lys Asn Gly Gly Pro Ser
   20 25 30
   Ser Gly Ala Pro Pro Pro Ser Lys
   35 40
<210> 242
   <211> 39
   <212> PRT
   <213> Exendin Analog 240
<400> 242
   His Gly Glu Gly Thr Phe Thr Ser Asp Leu Ser Lys Gln Leu Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 243
   <211> 39
   <212> PRT
   <213> Exendin Analog 241
<400> 243
   His Gly Glu Gly Thr Phe Thr Ser Asp Leu Ser Lys Gln Leu Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser
   35
<210> 244
   <211> 39
   <212> PRT
   <213> Exendin Analog 242
<400> 244
   His Gly Glu Gly Thr Phe Thr Ser Asp Leu Ser Lys Gln Leu Glu Glu
   1 5 10 15
   Glu Ala Val Lys Leu Phe Ile Glu Trp Leu Lys Asn Gly Gly Pro Ser
   20 25 30
   Ser Gly Ala Pro Pro Pro Ser
   35
<210> 245
   <211> 40
   <212> PRT
   <213> Exendin Analog 243
<400> 245
   His Gly Glu Gly Thr Phe Thr Ser Asp Leu Ser Lys Gln Leu Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Arg
   35 40
<210> 246
   <211> 39
   <212> PRT
   <213> Exendin Analog 244
<400> 246
   His Gly Glu Gly Thr Phe Thr Ser Asp Leu Ser Lys Gln Leu Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 247
   <211> 40
   <212> PRT
   <213> Exendin Analog 245
<400> 247
   His Gly Glu Gly Thr Phe Thr Ser Asp Leu Ser Lys Gln Leu Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Lys
   35 40
<210> 248
   <211> 40
   <212> PRT
   <213> Exendin Analog 246
<400> 248
   His Gly Glu Gly Thr Phe Thr Ser Asp Leu Ser Lys Gln Leu Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser Arg
   35 40
<210> 249
   <211> 39
   <212> PRT
   <213> Exendin Analog 247
<400> 249
   His Gly Glu Gly Thr Phe Thr Ser Asp Leu Ser Lys Gln Leu Glu Glu
   1               5                   10                  15
   Glu Ala Val His Leu Phe Ile Glu Trp Leu Lys Asn Gly Gly Pro Ser
               20                  25                  30
   Ser Gly Ala Pro Pro Pro Ser
           35
<210> 250
   <211> 40
   <212> PRT
   <213> Exendin Analog 248
<400> 250
   His Gly Glu Gly Thr Phe Thr Ser Asp Leu Ser Lys Gln Leu Glu Glu
   1               5                   10                  15
   Glu Ala Val His Leu Phe Ile Glu Trp Leu Lys Asn Gly Gly Pro Ser
               20                  25                  30
   Ser Gly Ala Pro Pro Pro Ser Lys
           35                  40
<210> 251
   <211> 40
   <212> PRT
   <213> Exendin Analog 249
<400> 251
   His Gly Glu Gly Thr Phe Thr Ser Asp Leu Ser Lys Gln Leu Glu Glu
   1               5                   10                  15
   Glu Ala Val Lys Leu Phe Ile Glu Trp Leu Lys Asn Gly Gly Pro Ser
               20                  25                  30
   Ser Gly Ala Pro Pro Pro Ser Arg
           35                  40
<210> 252
   <211> 39
   <212> PRT
   <213> Exendin Analog 250
<400> 252
   His Gly Glu Gly Thr Phe Thr Ser Asp Leu Ser Lys Gln Leu Glu Glu
   1               5                   10                  15
   Glu Ala Val Lys Leu Phe Ile Glu Trp Leu Lys Asn Gly Gly Pro Ser
   20 25 30
   Ser Gly Ala Pro Pro Pro Ser
   35
<210> 253
   <211> 40
   <212> PRT
   <213> Exendin Analog 251
<400> 253
   His Gly Glu Gly Thr Phe Thr Ser Asp Leu Ser Lys Gln Leu Glu Glu
   1 5 10 15
   Glu Ala Val Lys Leu Phe Ile Glu Trp Leu Lys Asn Gly Gly Pro Ser
   20 25 30
   Ser Gly Ala Pro Pro Pro Ser Lys
   35 40
<210> 254
   <211> 39
   <212> PRT
   <213> Exendin Analog 252
<400> 254
   His Gly Glu Gly Thr Phe Thr Ser Asp Leu Ser Lys Gln Ile Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 255
   <211> 39
   <212> PRT
   <213> Exendin Analog 253
<400> 255
   His Gly Glu Gly Thr Phe Thr Ser Asp Leu Ser Lys Gln Ile Glu Glu
   1 5 10 15
   Glu Ala Val His Leu Phe Ile Glu Trp Leu Lys Asn Gly Gly Pro Ser
   20 25 30
   Ser Gly Ala Pro Pro Pro Ser
   35
<210> 256
   <211> 39
   <212> PRT
   <213> Exendin Analog 254
<400> 256
   His Gly Glu Gly Thr Phe Thr Ser Asp Leu Ser Lys Gln Ile Glu Glu
   1 5 10 15
   Glu Ala Val Lys Leu Phe Ile Glu Trp Leu Lys Asn Gly Gly Pro Ser
   20 25 30
   Ser Gly Ala Pro Pro Pro Ser
   35
<210> 257
   <211> 40
   <212> PRT
   <213> Exendin Analog 255
<400> 257
   His Gly Glu Gly Thr Phe Thr Ser Asp Leu Ser Lys Gln Ile Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Arg
   35 40
<210> 258
   <211> 39
   <212> PRT
   <213> Exendin Analog 256
<400> 258
   His Gly Glu Gly Thr Phe Thr Ser Asp Leu Ser Lys Gln Ile Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 259
   <211> 40
   <212> PRT
   <213> Exendin Analog 257
<400> 259
   His Gly Glu Gly Thr Phe Thr Ser Asp Leu Ser Lys Gln Ile Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Lys
           35                  40
<210> 260
   <211> 40
   <212> PRT
   <213> Exendin Analog 258
<400> 260
   His Gly Glu Gly Thr Phe Thr Ser Asp Leu Ser Lys Gln Ile Glu Glu
   1               5                   10                  15
   Glu Ala Val His Leu Phe Ile Glu Trp Leu Lys Asn Gly Gly Pro Ser
               20                  25                  30
   Ser Gly Ala Pro Pro Pro Ser Arg
           35                  40
<210> 261
   <211> 39
   <212> PRT
   <213> Exendin Analog 259
<400> 261
   His Gly Glu Gly Thr Phe Thr Ser Asp Leu Ser Lys Gln Ile Glu Glu
   1               5                   10                  15
   Glu Ala Val His Leu Phe Ile Glu Trp Leu Lys Asn Gly Gly Pro Ser
               20                  25                  30
   Ser Gly Ala Pro Pro Pro Ser
           35
<210> 262
   <211> 40
   <212> PRT
   <213> Exendin Analog 260
<400> 262
   His Gly Glu Gly Thr Phe Thr Ser Asp Leu Ser Lys Gln Ile Glu Glu
   1               5                   10                  15
   Glu Ala Val His Leu Phe Ile Glu Trp Leu Lys Asn Gly Gly Pro Ser
               20                  25                  30
   Ser Gly Ala Pro Pro Pro Ser Lys
           35                  40
<210> 263
   <211> 40
   <212> PRT
   <213> Exendin Analog 261
<400> 263
   His Gly Glu Gly Thr Phe Thr Ser Asp Leu Ser Lys Gln Ile Glu Glu
   1               5                   10                  15
   Glu Ala Val Lys Leu Phe Ile Glu Trp Leu Lys Asn Gly Gly Pro Ser
               20                  25                  30
   Ser Gly Ala Pro Pro Pro Ser Arg
           35                  40
<210> 264
   <211> 39
   <212> PRT
   <213> Exendin Analog 262
<400> 264
   His Gly Glu Gly Thr Phe Thr Ser Asp Leu Ser Lys Gln Ile Glu Glu
   1               5                   10                  15
   Glu Ala Val Lys Leu Phe Ile Glu Trp Leu Lys Asn Gly Gly Pro Ser
               20                  25                  30
   Ser Gly Ala Pro Pro Pro Ser
           35
<210> 265
   <211> 40
   <212> PRT
   <213> Exendin Analog 263
<400> 265
   His Gly Glu Gly Thr Phe Thr Ser Asp Leu Ser Lys Gln Ile Glu Glu
   1               5                   10                  15
   Glu Ala Val Lys Leu Phe Ile Glu Trp Leu Lys Asn Gly Gly Pro Ser
               20                  25                  30
   Ser Gly Ala Pro Pro Pro Ser Lys
           35                  40
<210> 266
   <211> 65
   <212> DNA
   <213> Artificial Sequence
<220>
   <223> Chemically Synthesized
<400> 266
   aattccatgc acggcgaaac cttcaccagc gatctgagca aacagctgga agaagaagcg 60
   gttaa 65
<210> 267
   <211> 65
   <212> DNA
   <213> Artificial Sequence
<220>
   <223> Chemically Synthesized
<400> 267
   actgttcatc gaatggctga aaaacggcgg cccgagcagc ggcccgccgc cgccgagccg 60
   ttaga 65
<210> 268
   <211> 61
   <212> DNA
   <213> Artificial Sequence
<220>
   <223> Chemically Synthesized
<400> 268
   agcttctaac ggctcggcgg cggcgcgctg ctcgggccgc cgtttttcag ccattcgatg 60
   a 61
<210> 269
   <211> 69
   <212> DNA
   <213> Artificial Sequence
<220>
   <223> Chemically Synthesized
<400> 269
   acagtttaac cgcttcttct tccagctgtt tgctcagatc gctggtgaag gtgccttcgc 60
   cgtgcatgg 69